# EUROPEAN PATENT APPLICATION

(11) **EP 4 497 752 A1**
(43) Date of publication of application: **29.01.2025**
(21) Application number: 23773814.1
(22) Date of filing: 20.03.2023
(51) Int. Cl.: C07D 487/04, A61K 31/519, A61P 35/00

(54) **PYRIMIDO-PYRIDAZINONE COMPOUND AS TOLL-LIKE RECEPTOR AGONIST**

(30) Priority: 22.03.2022 CN 202210289208
(71) Applicant: Shanghai Visonpharma Co., Ltd., Shanghai 201321 (CN)
(72) Inventor: TANG, Guozhi, Shanghai 201321 (CN); MA, Dawei, Shanghai 201321 (CN); CHEN, Junli, Shanghai 201321 (CN); WANG, Yingyi, Shanghai 201321 (CN); LIU, Yongfu, Shanghai 201321 (CN)
(74) Representative: Cabinet Laurent & Charras
(86) International application number: PCT/CN2023/082607
(87) International publication number: WO 2023/179567

(57) **Abstract**

Provided are a compound as shown in formula I, and a preparation method therefor and a use thereof as a TLR7 and/or TLR8 agonist. The compound can be used for preparing a pharmaceutical composition for treating or preventing tumors or infections caused by viruses.

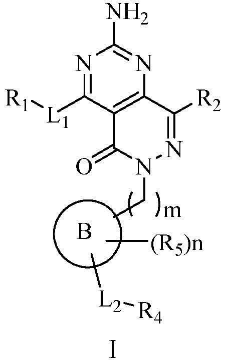

## Description

### Technical field

The present invention belongs to the field of pharmaceutical chemistry, in particular relates to pyrimido-pyridazinone compound as TLR7/8 agonist, and preparation methods therefor and applications thereof.

### Background

Toll-like receptors (TLRs) are a class of structurally conserved proteins that form the first barrier in the innate immune response. By recognising a variety of conserved pathogen-associated molecular patterns (PAMPs), TLRs can recognize endogenous molecules released after damage or non-physiological cell death of invasive microorganisms and tissue, and activate a signaling cascade, which lead to the production of pro-inflammatory cytokines. Inflammatory processes are critical for the onset and progression of a variety of diseases, such as type I diabetes, sepsis, cancer, and viral infectious diseases. Therefore, strategies to manipulate the inflammatory response through small-molecule TLRs modulators to treat related diseases are promising.

There are 10 known members of the family of human TLRs, which are type I transmembrane proteins characterized by a leucine-rich extracellular structural domain and a cytoplasmic tail containing the conserved Toll/ interleukin (IL)-1 receptor (TIR) structural domain. Within this family, TLR3, TLR7, TLR8 and TLR9 are located in the endosomal compartment.

Both TLR7 and TLR8 recognize RNA molecules (ssRNA) from single-stranded RNA viruses. TLR7 is expressed predominantly in plasmacytoid dendritic cells and B cells. TLR8 is predominantly found in mDCs, macrophages, and monocytes, and is also expressed in T cells. TLR7 stimulation primarily induces the production of type I interferons, including interferon-alpha (IFN-α), and causes the transcription of interferon-stimulated genes (ISGs). Interferon α is one of the main drugs used in the treatment of chronic hepatitis B or C. TLR8 is predominantly distributed in mDCs, macrophages, and monocytes, and is also expressed in T cells. TLR8 activation is mainly produces pro-inflammatory response and stimulates immune cells to secrete pro-inflammatory cytokines including tumor necrosis factor- (TNF-), IL-6 and other pro-inflammatory cytokines. The distribution of TLR7/8 overlaps with the downstream signaling pathway and therefore have similar function. TLR7/8 activation can exert direct antiviral activity through type I interferon response, and is also possible to promote the activation of NK and NKT cells by regulating innate immunity through pro-inflammatory responses, induce adaptive immunity, improve antigen presentation and activate dendritic cells, thereby enhances T-cell responses and promote the differentiation of B cells to produce antibodies. The development of TLR7/8 agonists has significant clinical value in both antiviral therapy and antitumor therapy, as well as in antibody-coupled drugs and vaccine adjuvants.

There are several relevant TLR7/8 agonist patent applications currently pending, but there is still a need for continued development of highly active, safer and therapeutically highly effective TLR7/8 agonists.

### Summary of the invention

The purpose of the present invention to provide a highly active, safer and therapeutically highly effective TLR7/8 agonist.

In a first aspect of the present invention, there is provided a compound of Formula I, or a solvate, a prodrug, a metabolite, or a pharmaceutically acceptable salt thereof, 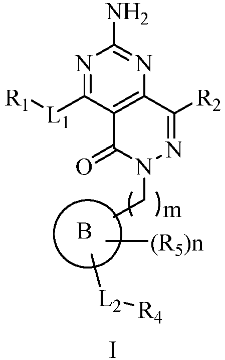 wherein:
L₁ is selected from the group consisting of: -O-, -NH-, -S-, -S(=O)- and -S(=O)₂-;
R₁ is selected from the group consisting of: H, C₁₋₁₂ alkyl, C₂₋₁₂ alkenyl, C₂₋₁₂ alkynyl, C₃₋₁₂ cycloalkyl, or 4-12-membered heterocycloalkyl; wherein R₁ may be further substituted by one or more R^{a} substituents, and R^{a} is selected from the group consisting of: hydrogen, halogen, hydroxyl, cyano, C₁₋₆ alkyl, C₁₋₆ alkoxy, C₃₋₆ cycloalkyl, -NR^{a1}R^{a2}, -NHC(=O)-R^{a3}, and one or more R^{a4}-substituted 5-6-membered heteroaryl;
R^{a1}, R^{a2}, R^{a3} and R^{a4} are selected from the group consisting of: C₁₋₆ alkyl, C₁₋₆ haloalkyl and C₃₋₆ cycloalkyl;
R₂ is independently selected from the group consisting of: hydrogen, halogen, cyano, C₁₋₆ alkyl, C₁₋₆ alkoxy, C₃₋₆ cycloalkyl and 4-6-membered heterocycloalkyl; wherein R₂ may be further substituted by one or more substituents selected from the group consisting of: halogen, hydroxy, cyano and amino;
m is 0, 1, 2, 3, 4, 5, 6, 7 or 8;
B is absent, or B is selected from the group consisting of: C₃₋₁₂ cycloalkyl, 4-12-membered heterocycloalkyl, C₆₋₁₂ aryl, 5-12-membered heteroaryl and 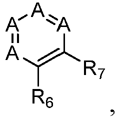 wherein each A is independently selected from C, CH and N, and R₆ and R₇ together with their attached carbon atoms form a C₄₋₇ cycloalkylidene or a C₄₋₇ heterocycloalkylidene, wherein one or more methylene groups in the C₄₋₇ cycloalkylidene or 4-7 membered heterocycloalkylidene may be each independently replaced by a carbonyl or a S(=O)₂; and heteroatoms in the 4-7 membered heterocycloalkylidene are selected from N, Oand S, and the number of heteroatoms is from 1 to 3;
L₂ is selected from the group consisting of: none, -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O)-, -S(=O)-and -S(=O)₂-; wherein R^{b} and R^{c} are selected from the group consisting of: hydrogen, halogen, C₁₋₆ alkyl, C₃₋₆ cycloalkyl, 4-6 membered heterocycloalkyl and C₁₋₆ haloalkyl, R^{d} is selected from the group consisting of: hydrogen, C₁₋₆ alkyl, C₃₋₆ cycloalkyl, 4-6 membered heterocycloalkyl, C₁₋₆ haloalkyl and hydroxyl-substituted C₁₋₆ alkyl, p is 0, 1, 2, 3, 4, 5 or 6, and q is 0 or 1;
R₄ is selected from the group consisting of: hydrogen, halogen, cyano, amino, hydroxy, C₁₋₁₂ alkyl, C₁₋₁₂ alkoxy, C₂₋₁₂ alkenyl, C₂₋₁₂ alkynyl, C₃₋₁₂ cycloalkyl, 4-12 membered heterocycloalkyl, C₆₋₁₂ aryland 5-12 membered heteroaryl; and R₄ is optionally substituted by one or more R^{e} substituents, wherein R^{e} is selected from the group consisting of: hydrogen, halogen, hydroxy, carboxylic acid, amino, C₁₋₆ alkyl, one or more R^{e1} substituted C₁₋₆ alkyl, C₁₋₆ alkoxy, C₁₋₆ haloalkoxy, C₃₋₁₂ cycloalkyl, 4-12 heterocycloalkyl, C₆₋₁₂ aryl, 5-12 membered heteroaryl, -N(R^{e2}R^{e3}), -C(=O)O-R^{e2}, -C(=O)NH-R^{e2} and -S(=O)₂-R^{e2},
and when L₂ is absent and R4 is H, B is selected from the group consisting of: C₃₋₁₂ cycloalkyl, 4-12 membered heterocycloalkyl, C₆₋₁₂ aryl, 5-12-membered heteroaryl and 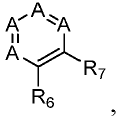 wherein each A is independently selected from C, CH and N, and R₆ and R₇ together with their attached carbon atoms form a C₄₋₇ cycloalkylidene or a C₄₋₇ heterocycloalkylidene;
R^{e1} is selected from the group consisting of: halogen and hydroxyl;
R^{e2} and R^{e3} are selected from the group consisting of: hydrogen, C₁₋₆ alkyl, C₃₋₆ cycloalkyl, C₁₋₆ haloalkyl and hydroxyl-substituted C₁₋₆ alkyl;
R₅ is selected from the group consisting of: halogen, hydroxyl, cyano, amino, C₁₋₆ alkyl, C₃₋₆ cycloalkyl, 4-6 membered heterocycloalkyl, C₁₋₆ haloalkyl, C₁₋₆ alkoxy, C₁₋₆ haloalkoxy, -C(=O)O-R^{f}, -C(=O)NH-R^{f} and -S(=O)₂-R^{f}; wherein R^{f} is selected from the group consisting of: hydrogen, C₁₋₆ alkyl, C₃₋₆ cycloalkyl and C₁₋₆ haloalkyl;
n is 1, 2, 3, 4, 5 or 6;
wherein each heterocyclyl may be saturated or partially unsaturated (but not having an aromatic structure), and in the heterocyclyl, the heteroatom is selected from N, O and S, and the number of heteroatoms is 1, 2, 3, or 4 (preferably 1 or 2); and in the heteroaryl, the heteroatom is selected from N, O and S, and the number of heteroatom is 1, 2, or 3.

In another preferred example, L₁ is selected from the group consisting of: -O-, -NH- and -S-.

In another preferred embodiment, L₁ is -NH-.

In another preferred embodiment, m is 1 or 2.

In another preferred embodiment, R₁ is selected from the group consisting of: C₁₋₁₂ alkyl, C₂₋₁₂ alkenyl, C₂₋₁₂ alkynyl, C₃₋₁₂ cycloalkyl and 4-12 membered heterocycloalkyl; and R₁ may be further substituted by one or more R^{a} substituents, and R^{a} is selected from the group consisting of: halogen, hydroxyl, cyano, C₁₋₆ alkyl, C₁₋₆ alkoxy and C₃₋₆ cycloalkyl.

In another preferred embodiment, R₁ is selected from the group consisting of: C₁₋₁₂ alkyl, C₂₋₁₂ alkenyl, C₂₋₁₂ alkynyl; and R₁ may be further substituted by one or more R^{a} substituents, and R^{a} is selected from the group consisting of: halogen, hydroxyl, cyano, C₁₋₆ alkyl, C₁₋₆ alkoxy and C₃₋₆ cycloalkyl.

In another preferred embodiment, R₁ is selected from the group consisting of: H, C₁₋₈ alkyl, C₁₋₈ alkoxy, C₂₋₈ alkenyl, C₂₋₈ alkynyl, C₃₋₈ cycloalkyl, and 4-8 membered heterocycloalkyl; and R₁ may be further substituted by one or more R^{a} substituents, and R^{a} is selected from the group consisting of: hydrogen, halogen, hydroxyl, cyano, C₁₋₄ alkyl and C₁₋₄ alkoxy.

In another preferred embodiment, R₁ is selected from the group consisting of: C₁₋₈ alkyl; and R₁ may be further substituted by one or more R^{a} substituents, and R^{a} is selected from the group consisting of: C₁₋₆ alkyl and C₃₋₆ cycloalkyl.

In another preferred embodiment, B is absent, or B is selected from the group consisting of: C₃₋₈ cycloalkyl, 4-7 membered heterocyclyl, a C₆₋₁₀ aryl and

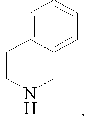

In another preferred embodiment, L₂ is selected from the group consisting of: -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O)-, -S(=O)- and-S(=O)₂-; wherein R^{b}, R^{c} are selected from the group consisting of: hydrogen, halogen and C₁₋₆ alkyl; R^{d} is hydrogen or C₁₋₆ alkyl; p is 0, 1, 2 or 3 and q is 0 or 1.

In another preferred embodiment, L₂ is selected from the group consisting of -(CH₂)ₚ-, -(CH₂)ₚ-NR^{d}-, -O-, -S-, -(CH₂)ₚ-C(=O)-, -(CH₂)ₚ-C(=O)NH- and -(CH₂)ₚ-NHC(=O)-; wherein R^{d} is hydrogen or C₁₋₆ alkyl; and p is 0, 1, 2 or 3.

In another preferred embodiment, R₄ is selected from the group consisting of: hydrogen, halogen, amino, hydroxyl, C₁₋₁₂ alkyl, C₁₋₁₂ alkoxy, C₂₋₁₂ alkenyl, C₂₋₁₂ alkynyl, C₃₋₁₀ cycloalkyl, 4-10 membered heterocycloalkyl, C₆₋₁₂ aryl and 5-12 membered heteroaryl; and R₄ may be optionally substituted by one or more R^{e} substituents; wherein R^{e} is selected from the group consisting of: hydrogen, halogen, hydroxyl, carboxylic acid, amino, C₁₋₆ alkyl, one or more R^{e1} substituted C₁₋₆ alkyl, C₁₋₆ alkoxy, C₁₋₆ haloalkoxy, C₃₋₈ cycloalkyl, 4-7 membered heterocycloalkyl, phenyl, 5-7 membered heteroaryl and -N(R^{e2}R^{e3}); wherein R^{e1} is selected from the group consisting of: halogen and hydroxyl; R^{e2} and R^{e3} are selected from the group consisting of: hydrogen, C₁₋₆ alkyl, C₃₋₆ cycloalkyl, C₁₋₆ haloalkyl or hydroxy-substituted C₁₋₆ alkyl.

In another preferred embodiment, R₄ is selected from the group consisting of: hydrogen, C₁₋₁₂ alkyl, C₁₋₁₂ alkoxy, cyclopropyl, cyclobutyl, cyclopentyl, cyclohexyl, and a group formed by losing one hydrogen atom from a ring selected from the group consisting of: 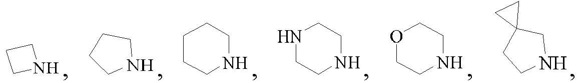 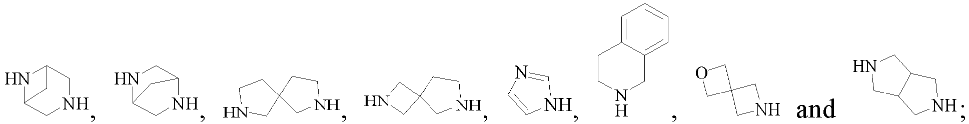 and R₄ may optionally be substituted by one or more R^{e} substituents.

In another preferred embodiment, R₅ is selected from the group consisting of: halogen, hydroxyl, cyano, amino, C₁₋₆ alkyl, C₁₋₆ alkoxy and C₃₋₆ cycloalkyl.

In another preferred embodiment, R₅ is selected from the group consisting of: halogen, C₁₋₄ alkyl and C₁₋₄ alkoxy.

In another preferred embodiment, the compound of formula I is compound I-1 to I-71.

In a second aspect of the present invention, there is provided a pharmaceutical composition, comprising: one or more of the compound of formula I as described in the first aspect of the present invention, a pharmaceutically usable salt thereof, a racemate, an R-isomer, an S-isomer, or a mixture thereof, and one or more pharmaceutically usable carriers, excipients, adjuvants, cofactors, and/or diluents.

In a third aspect of the present invention, there is provided the use of the compound of formula I as described in the first aspect of the present invention, a pharmaceutically usable salt thereof, a racemate, an R-isomer, an S-isomer, or a mixture thereof in the preparation of pharmaceutical compositions for the treatment or prevention of tumors or infections caused by viruses.

In another aspect, the present invention provides a complex, the complex being obtained by chemically bonding a compound as described in the present invention to a biosmall molecule or monoclonal antibodies.

It should be understood that, within the scope of the present invention, each of the above-described technical features of the present invention and each of the technical features specifically described below (e.g., example) can be combined with each other, thereby constituting a new or preferred technical solution. For the limitation of space, we will not repeat all of them herein.

### Detailed description of the invention

Through a long and intensive research, the present inventor has discovered a class of small molecule compounds having TLR7 and/or TLR8 agonistic activity, and the compounds described are structurally novel and have comparable or superior agonistic activity to similar compounds in the prior art. Based on the above discovery, the inventors have accomplished the present invention.

### Terms

In the present invention, the halogen is F, Cl, Br or I.

In the present invention, unless otherwise indicated, the terms used have the ordinary meaning known to those skilled in the art.

In the present invention, the term "C₁-C₆ alkyl" refers to straight or branched alkyl having 1 to 6 carbon atoms, and non-limitingly includes methyl, ethyl, propyl, isopropyl, butyl, isobutyl, sec-butyl, tert-butyl, pentyl, and hexyl, etc.; preferably ethyl, propyl, isopropyl, butyl, isobutyl, sec-butyl, and tert-butyl.

For the purposes of the present invention, the term "C₁-C₆ alkoxy" refers to straight or branched alkoxy having 1 to 6 carbon atoms, and non-limitingly includes methoxy, ethoxy, propoxy, isopropoxy, butoxy, and the like.

In the present invention, the term "C₃-C₇ cycloalkyl" refers to a cyclic alkyl having 3 to 7 carbon atoms on the ring, and non-limitably includes cyclopropyl, cyclobutyl, cyclopentyl, cyclohexyl, cycloheptyl, cyclooctyl, and the like. The terms "C₅-C₆ cycloalkyl" and "C₃-C₆ cycloalkyl" have similar meanings.

In the present invention, the terms "aryl ring" or "aryl" have the same meaning, and preferably "aryl" is "C₆-C₁₂ aryl" or "C₆-C₁₀ aryl". Preferably "aryl" is "C₆-C₁₂ aryl" or "C₆-C₁₀ aryl". The term "C₆-C₁₂ aryl" means an aromatic ring group having 6 to 12 carbon atoms, such as a phenyl, a naphthyl, or the like, which does not contain heteroatoms on the ring. The term "C₆-C₁₀ aryl" has a similar meaning.

In the present invention, the terms "aromatic heterocycle" or "heteroaryl" have the same meaning and refer to a heteroaromatic group comprising one to more heteroatoms. The heteroatoms referred to herein include oxygen, sulfur and nitrogen. Such as including furanyl, thiophenyl, pyridinyl, pyrazolyl, pyrrolyl, N-alkylpyrrolyl, pyrimidinyl, pyrazinyl, imidazolyl, tetrazolyl and the like. the heteroaryl ring may be thickened on an aryl, heterocyclyl or cycloalkyl ring, wherein the ring attached to the parent structure is a heteroaryl ring. The heteroaryl group may be optionally substituted or unsubstituted.

In the present invention, the term "3-9 membered carbocyclic group" refers to a 3-9 membered cyclic group in the form of a saturated or unsaturated (non-aromatic ring including monocyclic, bicyclic, spirocyclic, bridged, etc.) ring with a cyclo-skeletal structure comprising only carbon atoms, e.g., cyclopentyl, cyclohexyl, etc.

In the present invention, the term "3-9 membered heterocyclyl" refers to a saturated or unsaturated (non-aromatic ring, including monocyclic, bicyclic, spirocyclic, bridged ring, etc.) 3-9 membered cycloalkyl, such as dioxolane, etc., comprising 1 to 3 heteroatoms selected from among oxygen, sulfur and nitrogen on the ring. The term "3-7 membered heterocyclyl" has a similar meaning.

In the present invention, the term "substituted" refers to one or more hydrogen atoms on a specific group is substituted by a particular substituent. The particular substituent is a substituent described accordingly in the preceding text, or a substituent appearing in the respective examples. Unless specifically stated, a particular substituted group may have a substituent selected from a particular group at any substitutable site of the group, and the substituent may be the same or different at various positions. A cyclic substituent, such as a heterocycloalkyl, may be attached to another ring, such as a cycloalkyl, thereby forming a spiro-dicyclic ring system, e.g., where the two rings have a common carbon atom. It is to be understood by those skilled in the art that the combinations of substituents contemplated by the present invention are those that are stable or chemically realizable. The substituents are, for example (but not limited to): C₁₋₈ alkyl, C₂₋₈ alkenyl, C₂₋₈ alkynyl, C₃₋₈ cycloalkyl, 3- 12 membered heterocyclic, aryl, heteroaryl, halogen, hydroxyl, carboxy (-COOH), C₁₋₈ aldehyde, C₂₋₁₀ acyl, C₂₋₁₀ ester, amino, alkoxy, C₁₋₁₀ sulfonyl, and the like.

### Compounds of formula I with TLR7 and/or TLR8 modulating activity

The present invention provides a compound of Formula I, or a solvate, a prodrug, a metabolite, or a pharmaceutically acceptable salt thereof. 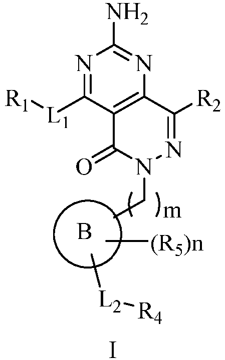 wherein each group is defined as described above.

### Preparation of compounds of formula I

The present invention also provided a method for preparing the above-described compounds as shown in Formula I. Specifically, the compounds of the present invention are prepared using the following process 1 or 2:

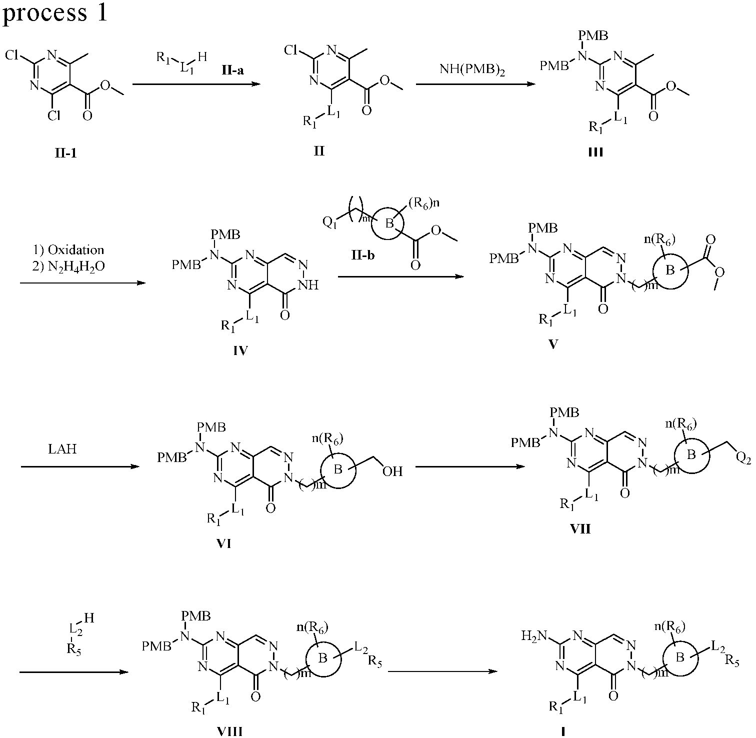

**In** process 1, starting compound II-1 undergoes a substitution reaction to obtain compound II; compound II further undergoes a substitution reaction to obtain compound III; compound III undergoes a oxidation reaction and a ring-closing reaction to obtain compound IV; compound IV undergoes a substitution reaction or a copper-catalyzed reaction to obtain compound V; compound V undergoes a ring-closing reaction to obtain compound VI; compound VI undergoes a substitution reaction to obtain compound VII; compound VII undergoes a substitution reaction with different amines to obtain compound VIII; and compound VIII undergoes deprotection to obtain compound I.

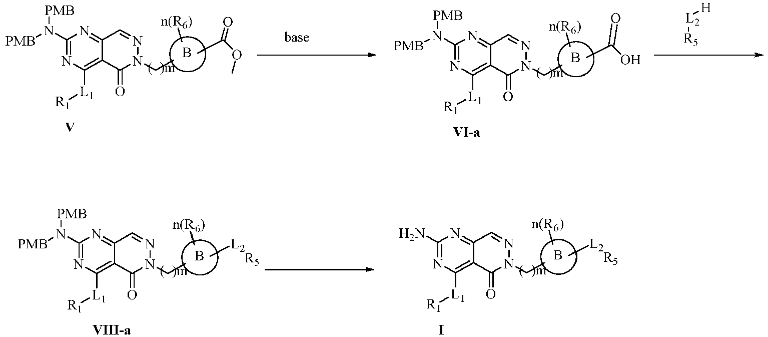

In process 2, compound V undergoes a hydrolysis reaction to obtain compound VI-a; compound VI-a undergoes a condensation reaction to obtain compound VIII-a; and compound VIII-a undergoes deprotection to obtain compound I.

### Pharmaceutical compositions containing the active ingredient

Because the compounds of the present invention have excellent agonistic activity of Toll-like receptors, the compounds of the present invention and their various crystalline forms, pharmaceutically acceptable inorganic or organic salts, hydrates or solvates, as well as pharmaceutical compositions comprising the compounds of the present invention as the main active ingredient can be used for preventing and/or treating (stabilizing, alleviating, or curing) the aberrant expression or activation of Toll-like receptors (in particular, TLR7, 8) associated diseases or conditions, such as tumors or infections caused by viruses; more preferably, said viruses are preferably one or more of HBV, HCV, HIV, and influenza viruses, and said tumors are preferably lung cancer, pancreatic cancer, renal cancer, head and neck cancer, breast cancer, lymphoma, skin cancer, uroepithelial cancer, gastric cancer, hepatocellular carcinoma, colorectal cancers, and the like.

The pharmaceutical compositions of the present invention comprise a compound of the present invention in a safe and effective amount and a pharmaceutically acceptable excipient or carrier. The term "safe and effective amount" means that the amount of the compound is sufficient to significantly improve the condition without causing serious side effects. Typically, the pharmaceutical compositions contain 0.01-99.99% by weight of the compound/dose of the present invention, preferably 0.1-99.9% of the compound/dose of the present invention. Preferably, said "dose" is a capsule or tablet.

"Pharmaceutically acceptable carrier" means: one or more compatibility solid or liquid fillers or gel substances which are suitable for human use and which must be of sufficient purity and sufficiently low toxicity. "Compatibility" herein refers to the ability of the components of the composition to be admixed with the compounds of the invention and with each other without appreciably reducing the efficacy of the compounds. Examples of pharmaceutically acceptable carrier portions are cellulose and derivatives thereof (e.g., sodium carboxymethyl cellulose, sodium ethyl cellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifiers (e.g., Tween^{®}), wetting agent (e.g. sodium dodecyl sulfate), coloring agent, flavoring agent, stabilizer, antioxidant, preservative, pyrogen-free water, etc.

There are no particular limitations on the mode of administration of the compounds or pharmaceutical compositions of the present invention, representative modes of administration include (but are not limited to): oral, parenteral (intravenously, intramuscularly, or subcutaneously).

Solid dosage forms for oral administration include capsules, tablets, pills, dispersions and granules. In these solid dosage forms, the active compound is mixed with at least one conventional inert excipient (or carrier), such as sodium citrate or dicalcium phosphate, or mixed with the following ingredients: (a) fillers or bulking agents, such as, starch, lactose, sucrose, dextrose, mannitol, and silicic acid; (b) binders, such as, hydroxymethyl cellulose, alginate, gelatin, polyvinylpyrrolidone, sucrose, and gum Arabic; (c ) humectants, such as, glycerin; (d) disintegrants, such as, agar, calcium carbonate, potato starch or tapioca starch, alginate, certain complex silicates, and sodium carbonate; (e) retardants, such as, paraffin waxes; (f) absorption accelerators, such as, quaternary amine compounds; (g) wetting agents, such as, cetearyl alcohols and glycerol monostearate; (h) adsorbents, such as, kaolin; and (i) lubricants, such as, talc, calcium stearate, magnesium stearate, solid polyethylene glycol, sodium dodecyl sulfate, or mixtures thereof. In capsules, tablets and pills, the dosage form may also contain a buffering agent.

Solid dosage forms such as tablets, sugar pills, capsules, pills and granules may be prepared using coatings and shell materials such as enteric coats and other materials well known in the art. They may comprise opacifying agents, and the release of the active compound or compounds in such compositions may be released in a delayed manner in a portion of the digestive tract. Examples of encapsulated components that may be employed are polymeric substances and wax-like substances. If necessary, the active compound may also be formed in the form of a microcapsule with one or more of the excipients mentioned above.

Liquid dosage forms for oral administration include pharmaceutically acceptable emulsions, solutions, suspensions, syrups or tinctures. In addition to the active compound, the liquid dosage form may comprise inert diluents routinely employed in the art, such as water or other solvents, solubilizers and emulsifiers, such as, ethanol, isopropanol, ethyl carbonate, ethyl acetate, propylene glycol, 1,3-butylene glycol, dimethylformamide, as well as oils, in particular, cottonseed oil, peanut oil, corn embryo oil, olive oil, castor oil, and sesame oil or mixtures of these substances, etc.

In addition to these inert diluents, the compositions may also comprise auxiliaries such as wetting agents, emulsifiers and suspending agents, sweeteners, flavoring agents and fragrances.

In addition to the active compounds, the suspensions may comprise suspending agents, such as, ethoxylated isooctadecanol, polyoxyethylene sorbitol and dehydrated sorbitan esters, microcrystalline cellulose, aluminum and agar in methanol or mixtures of these substances, etc.

Compositions for parenteral administration may comprise physiologically acceptable sterile aqueous or non-aqueous solutions, dispersions, suspensions, or emulsions, and sterile powders for re-dissolution into sterile injectable solutions or dispersions. Suitable aqueous and non-aqueous carriers, diluents, solvents or excipients include water, ethanol, polyols and suitable mixtures thereof.

The compounds of the present invention may be administered alone or in combination with other pharmaceutically acceptable compounds (e.g., anti-HBV agents).

When administered in combination, the pharmaceutical composition also includes a combination with one or more (2, 3, 4, or more) other pharmaceutically acceptable compounds. One or more (2, 3, 4, or more) of such other pharmaceutically acceptable compounds may be used concurrently, separately, or sequentially with the compounds of the present invention for the prevention and/or treatment of a disease or condition associated with the aberrant expression or activation of Toll-like receptors (in particular TLR7, 8).

The use of the pharmaceutical compositions is the application of a safe and effective amount of the compounds of the present invention to a mammal (e.g., a human) in need of treatment, wherein the dose at the time of administration is the dose that is considered pharmaceutically effective for administration. Of course, the specific dose should also take into account the route of administration, the health of the patient, and other factors, which are within the skill of the skilled practitioner.

The present invention is further described below in connection with specific examples. It should be understood that these examples are used only to illustrate the invention and are not intended to limit the scope of the invention. Experimental methods for which specific conditions are not indicated in the following embodiments are generally in accordance with conventional conditions, or in accordance with conditions recommended by the manufacturer. Percentages and portions are calculated by weight unless otherwise indicated.

In the following examples, the structures of the compounds were determined by nuclear magnetic resonance (NMR) or/and mass spectrometry (MS). The NMR shifts (δ) are given in units of 10-6 (ppm). The NMR determinations were made with a Bruker AVANCE-400 NMR instrument, the solvents for the determinations were deuterium dimethyl sulfoxide (DMSO-d6), deuterium chloroform (CDCl3), deuterium methanol ( CD3OD), and the internal standard was tetramethylsilane (TMS).

SHIMADZU LC system (column: Xselect^{®} CSHTM Prep-C18, 19 * 150 mm, liquid handler LH-40, pump LC-20AP, detector SPD-20A, system controller CBM-20A, solvent system: acetonitrile and 0.05% trifluoroacetic acid in water).

LC/MS spectra of the compounds were obtained using LC/MS (Agilent Technologies 1200 Series). The LC/MS conditions were as follows (run time 10 min):
Acidic conditions: A: 0.05% trifluoroacetic acid in water; B: 0.05% trifluoroacetic acid in acetonitrile;
Alkaline conditions: A: 0.05% NH₃-H₂O in water; B: acetonitrile
Neutral conditions: A: 10 mM NH₄OAC in water; B: acetonitrile
unless otherwise specified, in the following examples, the intermediates and final compounds are purified by using silica gel column chromatography, or by preparative HPLC on a reversed-phase column using Xselect^{®} CSHTM Prep-C18 (5 µm, OBDTM 19 * 150 mm) column or XBridgeTM Prep Phenyl (5 µm, OBDTM 30 * 100 mm).

Silica gel column chromatography generally used Yantai Huanghai silica gel 200-300 mesh silica gel as the carrier.

The CombiFlash Rapid Preparation Instrument used the CombiFlash Rf200 (TELEDYNE ISCO).

Thin Layer Chromatography (TLC) silica gel plate uses Yantai Yellow Sea HSGF254 or Qingdao GF254 silica gel plate, the size of the silica gel plate used for thin layer chromatography testing products is 0.15mm ~ 0.2mm, and the size of thin layer chromatography separation and purification products is 0.4mm ~ 0.5mm.

The known starting materials of the present invention can be synthesized using or according to methods known in the art or can be purchased from companies such as ABCR GmbH & Co.KG, Acros Organics, Aldrich Chemical Company, Shaoyuan Chemical Technology (Accela ChemBio Inc), Dare Chemicals and others.

Abbreviations: MeOH: methanol; DIEA: N,N-diisopropylethylamine; DMAP: 4-dimethylaminopyridine; DCM: dichloromethane; Dioxane: 1,4-dioxane; EA: ethyl acetate; Conc.HCl: concentrated hydrochloric acid; DMF: dimethylformamide; AcOH: acetic acid; DMA: dimethylacetamide; Xantphos: 4, 5-bis(diphenylphosphino)-9,9-dimethylxanthene; DCE: 1,2-dichloroethane; BH₃: borane; Boc₂O: tert-butyl tert-butoxycarbonyl carbonate; t-BuLi: tert-butyl lithium; DMF-DMA: 1,1-dimethoxy-N,N-dimethyl-methanamine; PPA: polyphosphoric acid; NaHMDS: sodium bis(trimethylsilyl)amide; POCl₃: phosphorous oxychloride; Pd/C: palladium carbon; ACN: acetonitrile; EtOH: ethanol; t-butyl nitrite: tert-butyl nitrite; Pyrrolidine: pyrrolidine; Toluene: toluene; TsOH: p-toluenesulfonic acid; Acrylamide: prop-2-enamide; THF: tetrahydrofuran; LDA: lithium diisopropyl Lithium Amino; LAH: Lithium Aluminum Hydrogen; Pd₂(dba)₃: tris(dibenzylideneacetone)dipalladium; PMB: p-methoxybenzyl; Xphos: 2-dicyclohexylphosphino-2,4,6-triisopropylbiphenyl; H₂: Hydrogen; Pd(dppf)Cl₂: [1,1'-bis(diphenylphosphino)ferrocene]palladium(II) dichloride; t-BuOK: Potassium Tertiary Butoxide; K₂CO₃ : potassium carbonate; HCOONH₄: ammonium formate; LiHMDS: lithium bis(trimethylsilyl)amide; Urea: urea; n-BuLi: n-butyllithium; MsCl: methanesulfonyl chloride; Dppf: bis(diphenylphosphino)ferrocene; Et₃N: triethylamine; AcCl: acetyl chloride; NaH: sodium hydride; TFA: trifluoroacetic acid; SOCl₂: sulfoxide chloride; NBS: N-bromosuccinimide; NCS: N-chlorosuccinimide; 1,4-Dioxane: 1,4-dioxane; K3PO4: Potassium Phosphate; RuPhos Pd G2: Chloro(2-dicyclohexylphosphino-2,6-diisopropoxy-1,1-biphenylyl)(2-amino-1,1-bi phenylyl-2-yl)palladium(II).

### Intermediate Int. A

### 2-(Bis(4-methoxybenzyl)amino)-4-(pentan-2-ylamino)pyrimido[4,5-d]pyri dazin-5(6H)-one

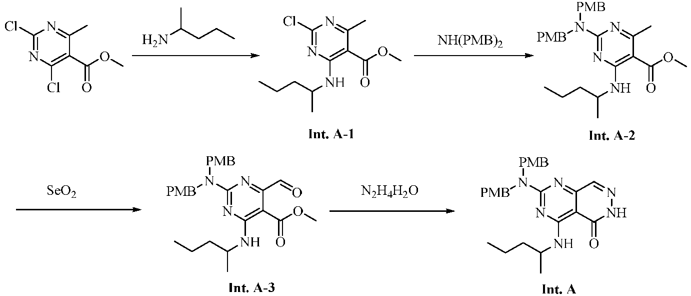

### Step 1: Preparation of methyl 2-chloro-4-methyl-6 -(pntane-2-ylamino) pyrimidine-5-carboxylate (Int. A-1)

Methyl 2,4-Dichloro-6-methylpyrimidine-5- carboxylate (500 mg, 2.262 mmol) and pentan-2-amine (237 mg, 2.71 mmol) were dissolved in DCM (10 mL), and DIEA (351 mg, 2.71 mmol) was added with stirring at 0 °C. The reaction mixture was continued to be stirred at 0°C for 2 hours. The reaction mixture was then poured into 10% potassium bicarbonate ice water and extracted with ethyl acetate. The organic phase was washed with saturated saline, dried over anhydrous sodium sulfate, filtered, and concentrated to obtain the crude product, which was purified by silica gel column chromatography to obtain a yellow solid methyl 2-chloro-4-methyl-6 -(pentan-2-ylamino) pyrimidine-5-carboxylate (560 mg, 91%). MS: 272.1[M+H]⁺

### Step 2: Preparation of methyl 2-(bis (4-methoxybenzyl) amino) -4-methyl-6 -(pentan-2-ylamino) pyrimidine-5-carboxylate (Int. A-2)

Methyl 2-chloro-4-methyl-6-(pentan-2-ylamino) pyrimidine-5-carboxylate (560 mg, 2.061 mmol) and bis(4-methoxybenzyl)amine (636 mg, 2.473 mmol) were dissolved in DMSO (10 mL) , and DIEA (400 mg, 3.09 mmol) was added with stirring at 0°C. The reaction mixture was continued to be stirred at 100 °C for 2 hours. The reaction mixture was then poured into ice water and extracted with ethyl acetate. The organic phase was washed with saturated brine, dried over anhydrous sodium sulfate, filtered, and concentrated to obtain the crude product, which was purified by silica gel column chromatography to obtain a yellow solid methyl 2-(bis (4-methoxybenzyl) amino) -4-methyl-6 -(pentan-2-ylamino) pyrimidine-5-carboxylate (800 mg, 78.8%). MS: 493.3 [M+H]⁺

### Step 3: Preparation of methyl 2-(bis (4-methoxybenzyl) amino) -4-formyl-6 -(pentan-2-ylamino) pyrimidine-5-carboxylate (Int. A-3)

2-(bis (4-methoxybenzyl) amino) -4-methyl-6 -(pentan-2-ylamino) pyrimidine-5-carboxylate (800 mg, 1.624 mmol) was dissolved in 1,4-Dioxane (5 mL) , and selenium dioxide (216 mg, 1.949 mmol) was added with stirring. The reaction mixture was continued with stirring at 100°C for 12 hours. The reaction mixture was then poured into ice water and extracted with ethyl acetate. The organic phase was washed with saturated brine, dried over anhydrous sodium sulfate, filtered, and concentrated to obtain the crude product, which was purified by silica gel column chromatography to obtain a yellow solid 2-(bis (4-methoxybenzyl) amino) -4-formyl-6 -(pentan-2-ylamino) pyrimidine-5-carboxylate (360 mg, 43.8%). MS: 507.2 [M+H]⁺

### Step 4: Preparation of 2-(bis (4-methoxybenzyl) amino)-4-(pentan-2-ylamino) pyrimidine [4, 5-d] pyridazine -5(6H) -one (Int. A)

methyl 2-(bis (4-methoxybenzyl) amino) -4-formyl-6 -(pentan-2-ylamino) pyrimidine-5-carboxylate (360 mg, 0.711 mmol) was dissolved in ethanol (5 mL), and hydrazine hydrate (45.5 mg, 1.421 mmol) was added with stirring at 0 °C. The reaction mixture was continued to be stirred at 85 °C for 1 hour. The reaction mixture was concentrated, filtered and washed with petroleum ether/ethyl acetate to obtain a white solid 2-(bis (4-methoxybenzyl) amino)-4-(pentan-2-ylamino) pyrimidine [4, 5-d] pyridazine -5(6H) -one (230 mg, 66.2%). MS: 489.2 [M+H]⁺

### Intermediate Int. B

### 2-(Bis(4-methoxybenzyl)amino)-4-((2-methoxyethyl)amino)pyrimido[4,5-d]p yridazin-5(6H)-one

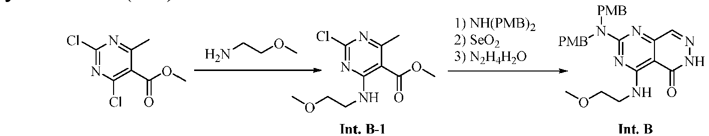

Intermediate Int. B was synthesized with reference to Intermediate Int. A by using 2-methoxyethane-1-amine instead of pentan-2-amine. MS: 477.2 [M+H]⁺

### Intermediate Int. C

### 2-(Bis(4-methoxybenzyl)amino)-4-(butylamino)pyrimido[4,5-d]pyridazin-5(6 H-one

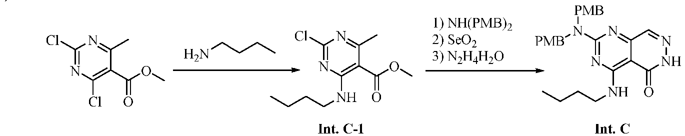

Intermediate Int. C was synthesized with reference to Intermediate Int. A by using n-butylamine instead of pentan-2-amine. MS: 475.2 [M+H]⁺

### Intermediate Int. D

### (R)-2-(Bis(4-methoxybenzyl)amino)-4-(pentan-2-ylamino)pyrimido[4,5-d]pyri dazin-5(6H)-one

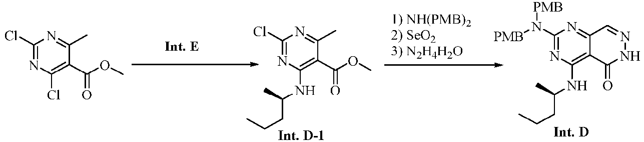

Intermediate Int. D was synthesized with reference to Intermediate Int. A by using Intermediate Int. E instead of pentan-2-amine. MS: 489.2 [M+H]⁺

### Intermediate Int. E

### (R)-Pentan-2-amine hydrochloride

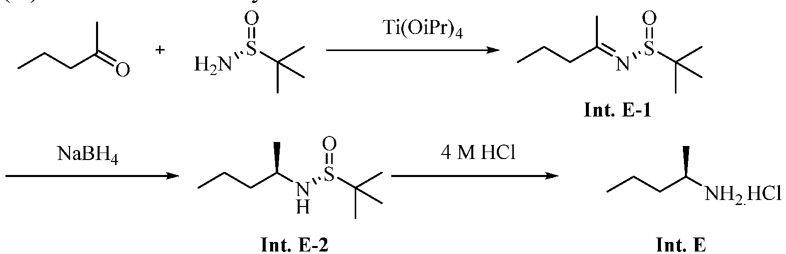

### Step 1: Preparation of (R,E)-2-Methyl-N-(2-pentylidene)propane-2-sulfinamide

Pentan-2-one (10 g, 116 mmol) was dissolved in EtOAc (200 mL) , then (R)-2-methylpropane-2-sulfinamide (14 g, 116 mmol) and tetraisopropyl titanate (66 g, 233 mmol) were sequentially added to the reaction mixture under stirring conditions at 10 °C. The reaction mixture was continued to react with stirring at 10 °C for 15 hours. The reaction mixture was then poured into ice water and extracted with ethyl acetate. The organic phase was washed with water and saturated saline respectively, dried over anhydrous sodium sulfate, filtered, and concentrated to obtain the crude product, which was purified by silica gel column chromatography to obtain the colorless oily intermediate Int. E-1 (7.0 g, 32%). MS: 190.2 [M+H]⁺

### Step 2: Preparation of (R)-2-Methyl-N-((R)-pentan-2-yl)propane-2-sulfinamide

Intermediate Int. E-1 (7.0 g, 37 mmol) was dissolved in THF (100 mL), sodium borohydride (2.1 g, 55.6 mmol) was added under stirring conditions at -70 °C and the reaction mixture was continued to be stirred at 20 °C for 2 hours. The reaction was then quenched by pouring ice water into the reaction mixture and extracted with ethyl acetate. The organic phase was washed with water and saturated saline respectively, dried over anhydrous sodium sulfate, filtered, and concentrated to obtain the crude product, which was purified by silica gel column chromatography to obtain the colorless oily intermediate Int. E-2 (3.5 g, 49%). MS: 192.2 [M+H]⁺

### Step 3: Preparation of (R)-Pentan-2-amine hydrochloride

Intermediate Int. E-2 (3.5 g, 18.3 mmol) in 4 N 1,4-dioxane hydrochloric acid solution (20 mL) was stirred at 20 °C for 12 hours. The reaction mixture was concentrated and evaporated to obtain the crude product, which was washed with ether to obtain the white solid intermediate Int. E(2.0g, 90%).

### Intermediate Int. F

### 2-(Bis(4-methoxybenzyl)amino)-4-(1-hydroxyhexan-3-yl)amino)pyrimido[4,5 -d]pyridazin-5(6H)-one

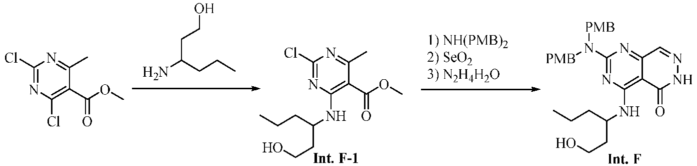

Intermediate Int. F was synthesized with reference to Intermediate Int. A by using 3-aminohexan-1-ol instead of pentan-2-amine. MS: 519.2 [M+H]⁺

### Intermediate Int. G

### (S)-Pentan-2-amine hydrochloride

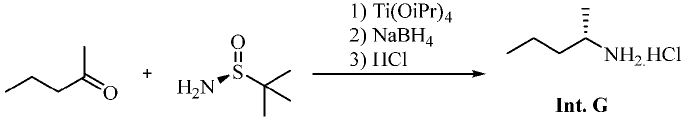

Intermediate Int. G was synthesized with reference to intermediate Int. E by using (S)-2-methylpropane-2-sulfinamide instead of (R)-2-methylpropane-2-sulfinamide.

### Intermediate Int. H

### (S)-2-(Bis(4-methoxybenzyl)amino)-4-(pentan-2-ylamino)pyrimido[4,5-d]p yridazin-5(6H)-one

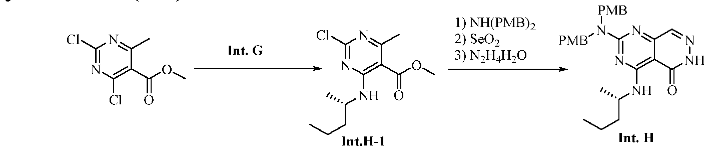

Intermediate Int. H was synthesized with reference to Intermediate Int. A by using Intermediate Int. G instead of pentan-2-amine. MS: 489.2 [M+H]⁺

### Intermediate Int. J

### 2-(Bis(4-methoxybenzyl)amino)-4-(butylamino)-8-methylpyrimido[4,5-d]p yridazin-5(6H)-one

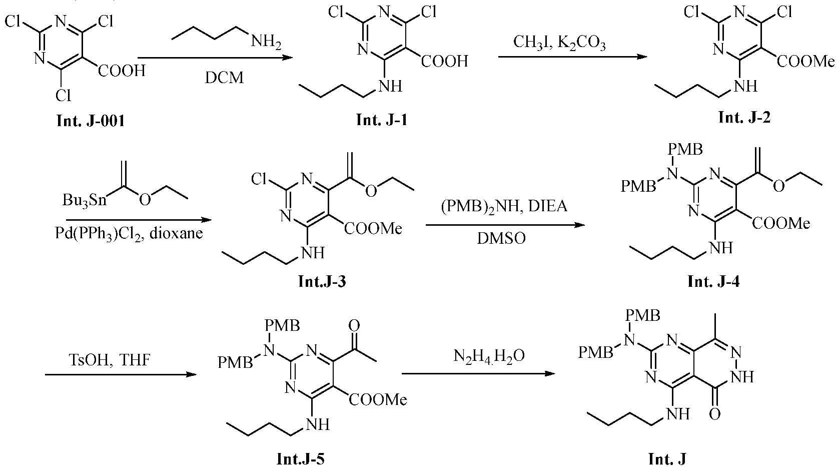

### Step 1: Preparation of the intermediate Int. J-1

Intermediate Int. J-001 (10 g, 44 mmol) and n-pentylamine (3.2 g, 44 mmol) were dissolved in DCM (200 mL), and DIEA (4.5 g, 44 mmol) was added with stirring at 0 °C. The reaction mixture was continued to be stirred at 0 °C for 2 hours. The reaction mixture was then poured into 10% potassium bicarbonate ice water and extracted with ethyl acetate. The organic phase was washed with saturated brine, dried over anhydrous sodium sulfate, filtered and concentrated to obtain the crude product which was purified by silica gel column chromatography to obtain a yellow solid intermediate Int. J-1 (9.5 g, 82%). MS: 264.1[M+H]⁺

### Step 2: Preparation of the intermediate Int. J-2

A reaction mixture of intermediate Int. J-1 (9 g, 34.1 mmol), potassium carbonate (7.1 g, 51.1 mmol) and iodomethane (5.8 g, 40.9 mmol) in DMF (100 ml) was stirred at 20 °C for 12 hours. The reaction mixture was then poured into 10% potassium bicarbonate ice water and extracted with ethyl acetate. The organic phase was washed with saturated brine, dried over anhydrous sodium sulfate, filtered and concentrated to obtain the crude product which was purified by silica gel column chromatography to obtain a yellow solid intermediate Int. J-2 (8 g, 84%). MS: 278.1[M+H]⁺

### Step 3: Preparation of the intermediate Int. J-3

A reaction mixture of intermediates Int. J-2 (1.7 g, 6.11 mmol), tributyl(1-ethoxyvinyl)tin (2.65 g, 7.33 mmol) and Pd(PPh3)2Cl2 (0.215 g, 0.306 mmol) in dioxane (20 mL) was reacted with stirring for 2 hours at 100 °C. The reaction mixture was then poured into 10% potassium bicarbonate ice water and extracted with ethyl acetate. The organic phase was washed with saturated brine, dried over anhydrous sodium sulfate, filtered and concentrated to obtain the crude product, which was purified by silica gel column chromatography to obtain the yellow solid intermediate Int. J-3 (1.3 g, 67.8%). MS: 314.1[M+H]⁺

### Step 4: Preparation of the intermediate Int. J-4

Intermediate Int. J-4 was synthesized with reference to Intermediate Int. A-2 by using Intermediate Int. J-3 instead of Int. A-1.

### Step 5: Preparation of the intermediate Int. J-5

A reaction mixture of intermediate Int. J-4 (1.7 g, 6.11 mmol) and TsOH (0.854 g, 4.49 mmol) in tetrahydrofuran (15 mL) was reacted with stirring at 50 °C for 2 hours. The reaction mixture was then poured into 10% potassium bicarbonate ice water and extracted with ethyl acetate. The organic phase was washed with saturated brine, dried over anhydrous sodium sulfate, filtered and concentrated to obtain the crude product, which was purified by silica gel column chromatography to obtain the yellow solid intermediate Int. J-5 (1.3 g, 86%). MS: 507.1[M+H]⁺

### Step 6: Preparation of the intermediate Int. J-6

Intermediate Int. J was synthesized with reference to Intermediate Int. A by using Intermediate Int. J-5 instead of Intermediate Int. A-3. MS: 489.2 [M+H]⁺

### Intermediate Int. K

### (S)-2-(Bis(4-methoxybenzyl)amino)-4-(1-hydroxypentan-2-yl)amino)pyrim ido[4,5-d]pyridazine-5(6H)-one

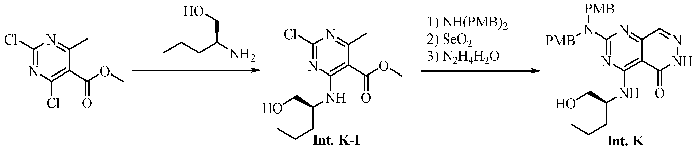

Intermediate Int. K was synthesized with reference to Intermediate Int. A by using (S)-2-aminopentan-1-ol instead of pentan-2-amine. MS: 505.2 [M+H]⁺

### Intermediate Int. K1

### (R)-2-(Bis(4-methoxybenzyl)amino)-4-(hexan-3-ylamino)pyrimido[4,5-d]p yridazin-5(6H)-one

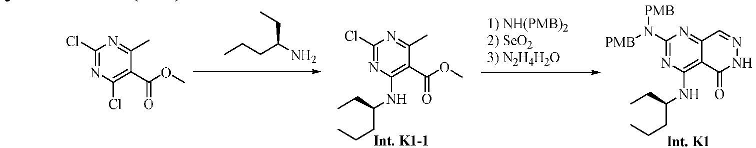

Intermediate Int. K1 was synthesized with reference to Intermediate Int. A by using (R)-hexan-3-amine instead of pentan-2-amine. MS: 503.2 [M+H]⁺

### Intermediate Int. K2

### (S)-2-(Bis(4-methoxybenzyl)amino)-4-(1-hydroxyhexan-2-yl)amino)pyrimi do[4,5-d]pyridazin-5(6H)-one

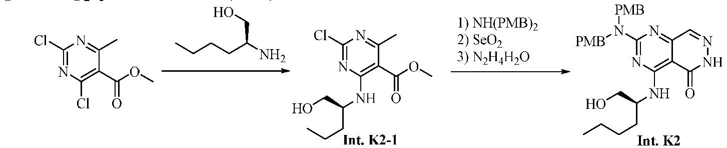

Intermediate Int. K2 was synthesized with reference to intermediate Int. A by using (S)-2-aminohexan-1-ol instead of pentan-2-amine. MS: 519.2 [M+H]⁺

### Intermediate Int. K3

### (R)-2-(Bis(4-methoxybenzyl)amino)-4-(1-hydroxypentan-2-yl)amino)pyri mido[4,5-d]pyridazin-5(6H)-one

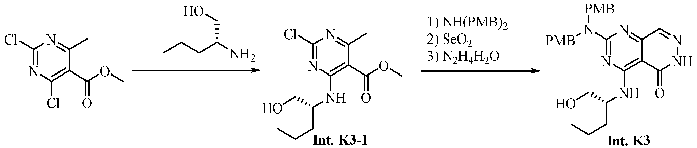

Intermediate Int. K3 was synthesized with reference to Intermediate Int. A by using (R)-2-aminopentan-1-ol instead of pentan-2-amine. MS: 505.2 [M+H]⁺

### Compound I-1

### 2-amino-4-(pentan-2-ylamino)-6-(4-(pyrrolidin-1-ylmethyl)benzyl)pyrimid o[4,5-d]pyridazin-5(6H)-one

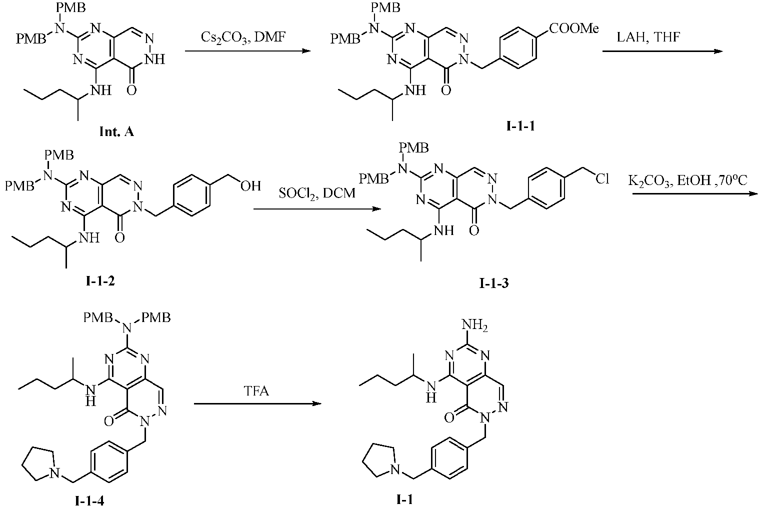

### Step 1: Preparation of 4-(2-(bis(4-methoxybenzyl)amino)-5-oxy-4-(pentan-2-ylamino)pyrimido[4,5-d] pyridazin-6(5H)-yl)methyl) methyl benzoate(Intermediate I-1-1)

Intermediate Int. A (230 mg, 0.471 mmol), methyl 4-(bromomethyl)benzoate (162 mg, 0.706 mmol), and Cs2CO3 (306 mg, 0.941 mmol) were dissolved in DMF (4 mL), and the reaction mixture was stirred and reacted for 2 hours at 100 °C. The reaction mixture was then poured into ice water and extracted with ethyl acetate. The organic phase was washed with water and saturated saline respectively, dried over anhydrous sodium sulfate, filtered and concentrated to obtain the crude product, which was purified by silica gel column chromatography to obtain the yellow solid intermediate I-1-1 (270 mg, 90%). MS: 637.2 [M+H]⁺

### Step 2: Preparation of 2-(Bis(4-methoxybenzyl)amino)-6-(4-(hydroxymethyl)benzyl)-4-(pentan-2-ylam ino)pyrimido[4,5-d]pyridazin-5(6H)-one(Intermediate I-1-2)

Intermediate I-1-1 (270 mg, 0.424 mmol) was dissolved in THF (5 mL), and 1.0 M LAH (0.5 mL, 0.5 mmol) in tetrahydrofuran was added under stirring at 0 °C. The reaction was carried out at 20 °C for 1 hour. The reaction mixture was continued to be stirred at 20 °C for 1 hour. The reaction mixture was poured then into ice water and extracted with ethyl acetate. The organic phase was washed with saturated brine, dried over anhydrous sodium sulfate, filtered, and concentrated to obtain the crude product, which was purified by silica gel column chromatography to obtain the white solid intermediate I-1-2 (198 mg, 77%). MS: 609.2 [M+H]⁺

### Step 3: Preparation of 2-(Bis(4-methoxybenzyl)amino)-6-(4-(chloromethyl)benzyl)-4-(pentan-2-ylamin o)pyrimido[4,5-d]pyridazin-5(6H)-one(Intermediate I-1-3)

Intermediate I-1-2 (198 mg, 0.326 mmol) was dissolved in DCM (1 mL), and sulfoxide chloride (48.6 mg, 0.408 mmol) was added with stirring at 0 °C. The reaction was continued for 1 hour at 20 °C. The reaction mixture was then concentrated to obtain a yellow solid Intermediate I-1-3 (245 mg, 95%). MS: 628.2 [M+H]⁺

### Step 4: Preparation of 2-(Bis(4-methoxybenzyl)amino)-4-(pentan-2-ylamino)-6-(4-(pyrrolidin-1-ylmet hyl)benzyl)pyrimido[4,5-d]pyridazin-5(6H)-one(Intermediate I-1-4)

Intermediate I-1-3 (163 mg, 0.259 mmol) and pyrrolidine (36.9 mg, 0.518 mmol) were dissolved in EtOH (1 mL), and K₂CO₃ (71.6 mg, 0.518 mmol) was added with stirring at 20 °C. The reaction mixture was continued to be stirred at 85 °C for 1 hour. The reaction mixture was then poured into ice water and extracted with ethyl acetate. The organic phase was washed with saturated brine, dried over anhydrous sodium sulfate, filtered, and concentrated to obtain the crude product, which was purified by silica gel column chromatography to obtain the white solid intermediate I-1-4 (140 mg, 80.2%). MS: 662.2 [M+H]⁺.

### Step 5: Preparation of 2-Amino-4-(pentan-2-ylamino)-6-(4-(pyrrolidin-1-ylmethyl)benzyl)pyrimido[4, 5-d]pyridazin-5(6H)-one(Intermediate I-1)

Intermediate I-1-4 (140 mg, 0.212 mmol) was dissolved in trifluoroacetic acid (20 mL), and the reaction mixture was stirred at 70 °C for 2 hours. The reaction mixture was then concentrated and evaporated to dryness and extracted with ethyl acetate. The organic phase was washed with saturated sodium bicarbonate solution and saturated brine, dried over anhydrous sodium sulfate, filtered, and concentrated to obtain the crude product, which was purified by Prep-HPLC to obtain a white solid compound I-1 (78 mg, 87%). MS: 422.2 [M+H]⁺. ¹H NMR (400 MHz, DMSO-*d₆*) δ 8.90 (d, *J =* 8.3 Hz, 1H), 7.83 (s, 1H), 7.28-7.19 (m, 4H), 7.02 (br, 2H), 5.24-5.07 (m, 2H), 4.25-4.16 (m, 1H), 3.54 (s, 2H), 2.43-2.38 (m, 4H), 1.68-1.64 (m, 4H), 1.54-1.46 (m, 2H), 1.37-1.27 (m, 2H), 1.16 (d, *J =* 6.5 Hz, 3H), 0.88 (t, *J =* 7.3 Hz, 3H).

### Compound I-1a and Compound I-1b

### (R)-2-amino-4-(pentan-2-ylamino)-6-(4-(pyrrolidin-1-ylmethyl)benzyl)pyr imido[4,5-d]pyridazin-5(6H)-one and (S)-2-Amino-4-(pentan-2-ylamino)-6-(4-(pyrrolidin-1-ylmethyl)benzyl)pyrimid o[4,5-d]pyridazin-5(6H)-one

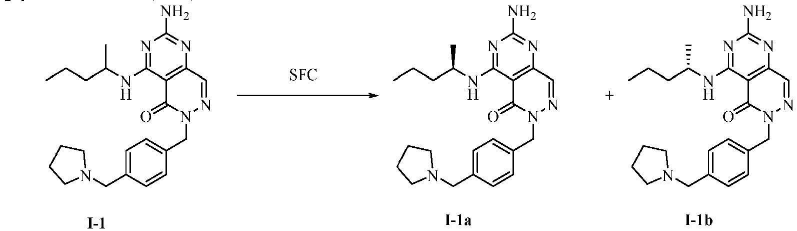

Compound I-1 was chirally split by SFC (column model: CHIRALPAK IG, 2 cm × 25 cm, 5 µm column, mobile phase: 20% ethanol in carbon dioxide) to obtain the first component peak Compound I-1a (retention time 4.65 min) and the second component peak Compound I-1b (retention time 5.39 min), which both were white solids.

**Compound I-1a:** MS: 422.2 (M+H)⁺.¹H NMR (400 MHz, DMSO-*d₆*) δ 8.89 (d, J = 8.3 Hz, 1H), 7.82 (s, 1H), 7.28-7.16 (m, 4H), 7.02 (br, 2H), 5.24-5.04 (m, 2H), 4.19 (q, J = 7.0 Hz, 1H), 3.50 (s, 2H), 2.42-2.31 (m, 4H), 1.70-1.60 (m, 4H), 1.55-1.44 (m, 2H), 1.37-1.26 (m, 2H), 1.15 (d, J = 6.5 Hz, 3H), 0.87 (t, J = 7.3 Hz, 3H)

**Compound I-1b:** MS: 422.2 (M+H)⁺.¹H NMR (400 MHz, DMSO-*d₆*) δ 8.89 (d, J = 8.3 Hz, 1H), 7.82 (s, 1H), 7.28-7.16 (m, 4H), 7.02 (br, 2H), 5.24-5.04 (m, 2H), 4.19 (q, J = 7.0 Hz, 1H), 3.50 (s, 2H), 2.42-2.31 (m, 4H), 1.70-1.60 (m, 4H), 1.55-1.44 (m, 2H), 1.37-1.26 (m, 2H), 1.15 (d, J = 6.5 Hz, 3H), 0.87 (t, J = 7.3 Hz, 3H)

### Compound I-2

### 2-amino-4-(butylamino)-6-(4-(piperazine-1-carbonyl)benzyl)pyrimido[4,5-d]pyridazin-5(6H)-one

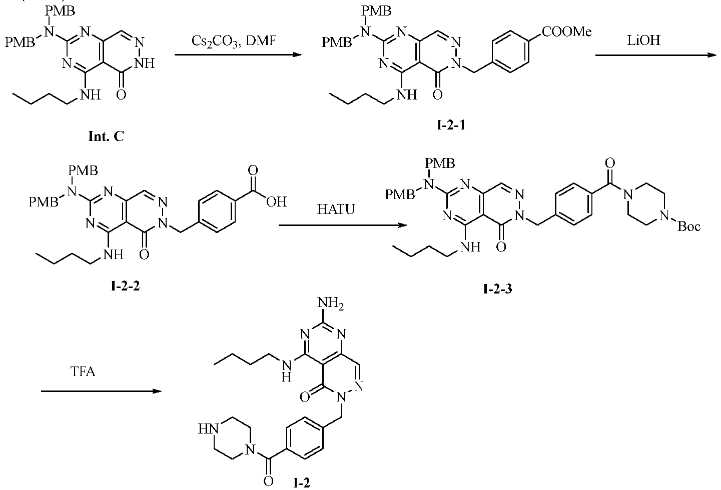

### Step 1: Preparation of 4-((2-(bis(4-methoxybenzyl)amino)-4-(butylamino)-5-oxopyridino[4,5-d]pyrida zin-6(5H)-yl)methyl) methyl benzoate(Intermediate I-2-1)

**Intermediate I-2-1 was synthesized with reference to Intermediate I-1-1by using Intermediate Int. C instead of Intermediate Int. A. MS: 623.3 [M+H]⁺.**

### Step 2: Preparation of 4-((2-(Bis(4-methoxybenzyl)amino)-4-(butylamino)-5-oxopyridino[4,5-d]pyrida zin-6(5H)-yl)methyl)benzoic acid(Intermediate I-2-2)

Intermediate I-2-1 (500 mg, 0.803 mmol) was dissolved in THF (5 mL) and H₂O (5 mL), and lithium hydroxide (160 mg, 4.01 mmol) was added with stirring at 0 °C. The reaction mixture was continued to be stirred at 20 °C for 1 hour. The reaction mixture was adjusted to pH 5 with 1N hydrochloric acid and extracted with ethyl acetate. The organic phase was washed with saturated saline, dried over anhydrous sodium sulfate, filtered, and concentrated to obtain a crude product, which was purified by silica gel column chromatography to obtain a yellow solid intermediate I-2-2 (400 mg, 82%). MS: 609.3 [M+H]⁺

### Step 3: Preparation of tert-butyl 4-(4-((2-(Bis(4-methoxybenzyl)amino)-4-(butylamino)-5-oxopyridino[4,5-d]pyri dazin-6(5H)-yl)methyl)benzoyl)piperazine-1-carboxylate (Intermediate I-2-3)

A mixed solution of intermediate I-2-2 (80 mg, 0.131 mmol), HATU (75.0 mg, 0.197 mmol), DIEA (34.0 mg, 0.263 mmol) and tert-butyl piperazine-1-carboxylate (49.0 mg, 0.263 mmol) in DMF (2 ml) was reacted at 20 °C with continued stirring for 2 hours. The reaction mixture was then poured into ice water and extracted with ethyl acetate. The organic phase was washed with saturated brine, dried over anhydrous sodium sulfate, filtered and concentrated to obtain a crude product, which was purified by silica gel column chromatography to obtain a yellow solid intermediate I-2-3 (80 mg, 78%). MS: 777.4 [M+H]⁺

### Step 4: Preparation of 2-amino-4-(butylamino)-6-(4-(piperazine-1-carbonyl)benzyl)pyrimido[4,5-d]py ridazin-5(6H)-one(Intermediate I-2)

Compound I-2 was synthesized with reference to Compound I-1 by using Intermediates I-2-3 instead of Intermediates I-1-4 to obtain white solid Compound I-2. MS: 437.2 [M+H]⁺.¹H NMR (400 MHz, DMSO-*d₆*) δ 8.97 (t, *J =* 5.7 Hz, 1H), 7.85 (s, 1H), 7.37-7.31 (m, 4H), 7.05 (s, 2H), 5.22 (s, 2H), 3.62-3.52 (m, 2H),3.45-3.40 (m, 2H), 3.30-3.20 (m, 2H), 2.82-2.67 (m, 4H), 1.54 (q, *J =* 7.2 Hz, 2H), 1.34 (q, *J =* 7.4 Hz, 2H), 0.90 (t, *J =* 7.3 Hz, 3H).

### Compound I-3

### 2-amino-4-(butylamino)-6-(4-(4-methylpiperazine-1-carbonyl)benzyl)pyri mido[4,5-d]pyridazin-5(6H)-one

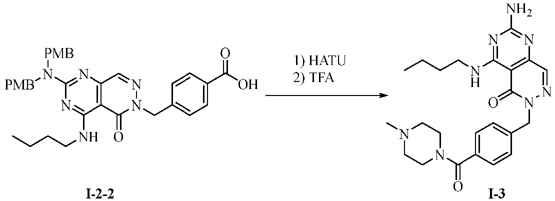

Compound I-3 was synthesized with reference to Compound I-2 by using 1-methylpiperazine instead of tert-butyl piperazine-1-carboxylate to obtain white solid Compound I-3. MS: 451.2 [M+H]⁺.¹H NMR (400 MHz, DMSO-*d₆*) δ 8.97 (t, J = 5.7 Hz, 1H), 7.85 (s, 1H), 7.36-7.30 (m, 4H), 7.05 (s, 2H), 5.22 (s, 2H), 3.45-3.40 (m, 2H), 3.35-3.25 (m, 4H), 2.40-2.20 (m, 4H), 2.18 (s, 3H), 1.57-1.51 (m, 2H), 1.39-1.29 (m, 2H), 0.90 (t, J = 7.3 Hz, 3H).

### Compound I-4

### 2-amino-4-((2-methoxyethyl)amino)-6-(4-(pyrrolidin-1-ylmethyl)benzyl)py rimido[4,5-d]pyridazin-5(6H)-one

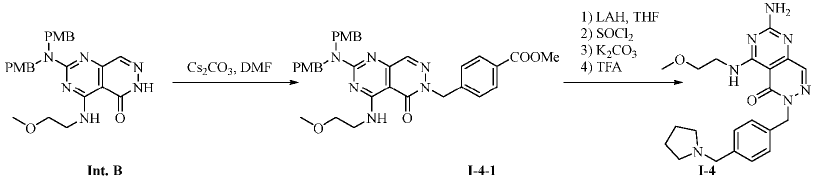

Compound I-4 was synthesized with reference to Compound I-1 by using Intermediate Int. B instead of Intermediate Int. A to obtain white solid Compound I-4. MS: 410.2 [M+H]⁺.¹H NMR (400 MHz, DMSO-*d₆*) δ 9.07 (t, J = 5.5 Hz, 1H), 7.84 (s, 1H), 7.24 (q, J = 8.3 Hz, 4H), 7.06 (br, 2H), 5.17 (s, 2H), 3.62-3.58 (m, 2H), 3.55 (s, 2H), 3.52-3.48 (m, 2H), 3.28 (s, 3H), 2.44-2.39 (m, 4H), 1.69-1.65 (m, 4H).

### Compound I-5

### 2-amino-4-(butylamino)-6-(4-(pyrrolidin-1-ylmethyl)benzyl)pyrimido[4,5-d]pyridazin-5(6H)-one

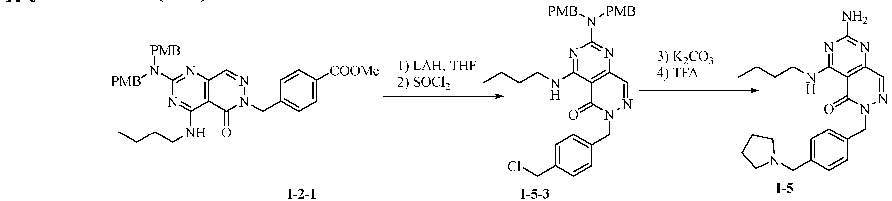

Compound I-5 was synthesized with reference to Compound I-1 by using Intermediate I-2-1 instead of Intermediate I-1-1 to obtain white solid Compound I-5. MS: 408.2 [M+H]⁺.¹H NMR (400 MHz, DMSO-*d₆*) δ 7.84 (s, 1H), 7.24 (q, J = 8.0 Hz, 4H), 7.03 (s, 2H), 5.17 (s, 2H), 3.56 (s, 3H), 3.43 (q, J = 6.6 Hz, 2H), 2.46-2.39 (m, 4H), 1.71-1.66 (m, 4H), 1.58-1.51 (m, 2H), 1.37-1.32 (m, 2H), 0.91 (t, J = 7.4 Hz, 3H).

### Compound I-6

### 2-amino-4-(butylamino)-6-(4-(morpholinomethyl)benzyl)pyrimido[4,5-d]p yridazin-5(6H)-one

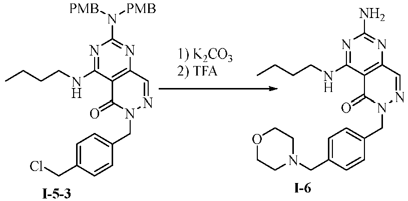

Compound I-6 was synthesized with reference to Compound I-5 by using morpholine instead of pyrrolidine to obtain white solid Compound I-6. MS: 424.2 [M+H]⁺.¹H NMR (400 MHz, DMSO-*d₆*) δ 8.99 (t, J = 5.7 Hz, 1H), 7.83 (s, 1H), 7.28-7.20 (m, 4H), 7.00 (br, 2H), 5.16 (s, 2H), 3.56-3.51 (m, 4H), 3.45-3.39 (m, 4H), 2.32-2.27 (m, 4H), 1.56-1.49 (m, 2H), 1.38-1.28 (m, 2H), 0.90 (t, J = 7.4 Hz, 3H).

### Compound I-7

### 2-amino-4-(butylamino)-6-(4-((dimethylamino)methyl)benzyl)pyrimido[4, 5-d]pyridazin-5(6H)-one

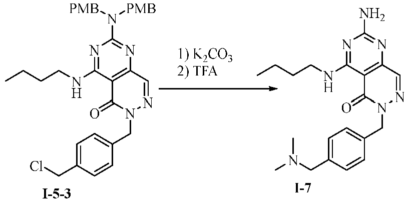

Compound I-7 was synthesized with reference to Compound I-5 by using dimethylamine hydrochloride instead of pyrrolidine to obtain white solid Compound I-7. MS: 382.2 [M+H]⁺.¹H NMR (400 MHz, DMSO-*d₆*) δ 8.99 (t, J = 5.7 Hz, 1H), 7.83 (s, 1H), 7.24-7.20 (m, 4H), 7.04 (br, 2H), 5.16 (s, 2H), 3.45-3.38 (m, 2H), 2.32 (s, 2H), 2.09 (s, 6H), 1.58-1.49 (m, 2H), 1.38-1.28 (m, 2H), 0.90 (t, J = 7.3 Hz, 3H).

### Compound I-8

### 2-amino-4-(butylamino)-6-((1,2,3,4-tetrahydroisoquinolin-6-yl)methyl)pyr imido[4,5-d]pyridazin-5(6H)-one

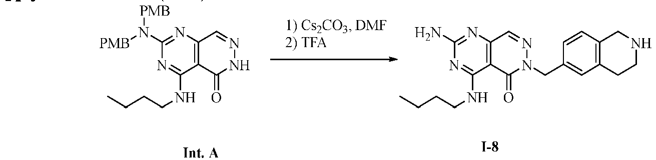

Compound I-8 was synthesized with reference to Compound I-2 by using tert-butyl 6-(bromomethyl)-3,4-dihydroisoquinoline-2(1H)- carboxylate instead of methyl 4-(bromomethyl) benzoate to obtain white solid compound I-8. MS: 380.2 [M+H]⁺.¹H NMR (400 MHz, DMSO-*d₆*) δ 8.98 (t, J = 5.6 Hz, 1H), 8.25 (s, 1H), 7.82 (s, 1H), 7.09-7.02 (m, 5H), 5.12 (s, 2H), 4.00 (s, 2H), 3.42 (q, J = 6.8 Hz, 2H), 3.10 (t, J = 6.1 Hz, 2H), 2.77 (t, J = 6.1 Hz, 2H), 1.58-1.50 (m, 2H), 1.39-1.28 (m, 2H), 0.90 (t, J = 7.3 Hz, 3H).

### Compound I-9

### 2-amino-4-(butylamino)-6-(4-(piperazin-1-ylmethyl)benzyl)pyrimido[4,5-d]py ridazin-5(6H)-one

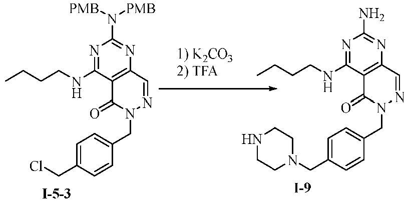

Compound I-9 was synthesized with reference to Compound I-5 by using tert-butyl piperazine-1-carboxylate instead of pyrrolidine to obtain white solid Compound I-9. MS: 423.2 [M+H]⁺.¹H NMR (400 MHz, DMSO-*d₆*) δ 8.98 (t, J = 5.6 Hz, 1H), 8.32 (s, 1H), 7.83 (s, 1H), 7.26-7.20 (m, 4H), 7.04 (br, 2H), 5.16 (s, 2H), 3.45-3.39 (m, 4H), 2.84-2.79 (m, 4H), 2.40-2.35 (m, 4H), 1.58-1.49 (m, 2H), 1.39-1.28 (m, 2H), 0.90 (t, J = 7.4 Hz, 3H).

### Compound I-10

### 2-amino-4-(butylamino)-6-(4-((cyclopropylamino)methyl)benzyl)pyrimido [4,5-d]pyridazin-5(6H)-one

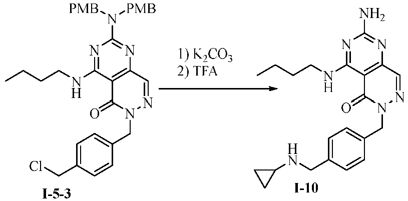

Compound I-10 was synthesized with reference to Compound I-5 by using cyclopropylamine instead of pyrrolidine to obtain white solid Compound I-10. MS: 394.2 [M+H]⁺.¹H NMR (400 MHz, DMSO-*d₆*) δ 8.99 (t, J = 5.6 Hz, 1H), 7.82 (s, 1H), 7.28-7.16 (m, 4H), 7.00 (br, 2H), 5.15 (s, 2H), 3.66 (s, 2H), 3.45-3.40 (m, 2H), 2.02-1.98 (m, 1H), 1.58-1.50 (m, 2H), 1.38-1.28 (m, 2H), 0.90 (t, J = 7.3 Hz, 3H), 0.34-0.28 (m, 2H), 0.24 -0.19 (m, 2H).

### Compound I-11

### 6-(4-((3,6-Diazabicyclo[3.1.1]heptan-3-yl)methyl)benzyl)-2-amino-4-(butyl amino)pyrimido[4,5-d]pyridazin-5(6H)-one

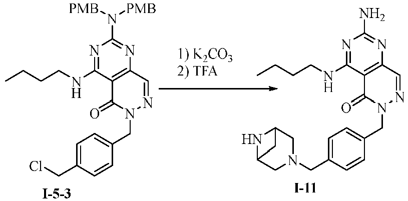

Compound I-11 was synthesized with reference to Compound I-5 by using tert-butyl 3,6-diazacyclo[3.1.1]heptane-6- carboxylate instead of pyrrolidine to obtain white solid compound I-11. MS: 435.2 [M+H]⁺.¹H NMR (400 MHz, DMSO-*d₆*) δ 8.99 (t, J = 5.6 Hz, 1H), 8.37 (s, 1H), 7.83 (s, 1H), 7.31-7.21 (m, 4H), 7.03 (br, 2H), 5.17 (s, 2H), 3.81 (d, J = 5.7 Hz, 2H), 3.68 (s, 2H), 3.45-3.40 (m, 2H), 3.04-2.98 (m, 2H), 2.78-2.74 (m, 2H), 2.44-2.39 (m, 1H), 1.97 (d, J = 8.4 Hz, 1H), 1.58-1.50 (m, 2H), 1.39-1.28 (m, 2H), 0.90 (t, J = 7.3 Hz, 3H).

### Compound I-12

### 6-(4-((2,6-Diazaspiro[3.4]octan-6-yl)methyl)benzyl)-2-amino-4-(butylamin o)pyrimido[4,5-d]pyridazin-5(6H)-one

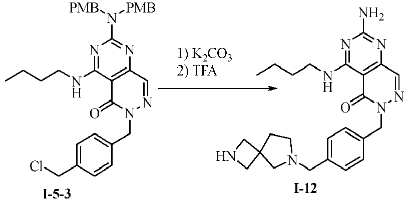

Compound I-12 was synthesized with reference to compound I-5, and was prepared by using tert-butyl 2,6-diazaspiro[3.4]octane-2- carboxylate instead of pyrrolidine to obtain a white solid compound I-12. MS: 449.2 [M+H]⁺. ¹H NMR (400 MHz, DMSO-*d₆*) δ 8.98 (t, J = 5.6 Hz, 1H) 8.60 (br, 1H), 7.85 (s, 1H), 7.30-7.22 (m, 4H), 7.05 (br, 2H), 5.18 (s, 2H), 3.92-3.87 (m, 2H), 3.85-3.79 (m, 2H), 3.70-3.58 (m, 2H), 3.43 (q, J = 6.6 Hz, 2H), 2.80-2.55 (m, 4H), 2.08-2.02 (m, 2H), 1.59 -1.50 (m, 2H), 1.39-1.29 (m, 2H), 0.90 (t, J = 7.3 Hz, 3H).

### Compound I-13

### 2-amino-4-(butylamino)-6-((2-isopropyl-1,2,3,4-tetrahydroisoquinolin-6-yl )methyl)pyrimido[4,5-d]pyridazin-5(6H)-one

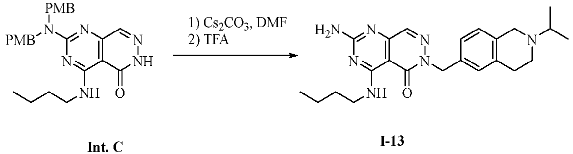

Compound I-13 was synthesized with reference to Compound I-2 by using 6-(bromomethyl)-2-isopropyl-1,2,3,4-tetrahydroisoquinoline instead of methyl 4-(bromomethyl)benzoate to obtain white solid compound I-13. MS: 422.2 [M+H]⁺.¹H NMR (400 MHz, DMSO-*d₆*) δ 8.99 (t, J = 5.7 Hz, 1H), 7.80 (s, 1H), 7.05-6.95 (m, 5H), 5.10 (s, 2H), 3.61 (s, 2H), 3.45-3.39 (m, 2H), 2.90-2.81 (m, 1H), 2.76-1.71 (m, 2H), 2.70-2.65 (m, 2H), 1.58-1.50 (m, 2H), 1.39-1.29 (m, 2H), 1.03 (d, J = 6.5 Hz, 6H), 0.90 (t, J = 7.3 Hz, 3H).

### Compound I-14

### 2-amino-4-(butylamino)-6-(2-methoxy-4-(pyrrolidin-1-ylmethyl)benzyl)py rimido[4,5-d]pyridazin-5(6H)-one

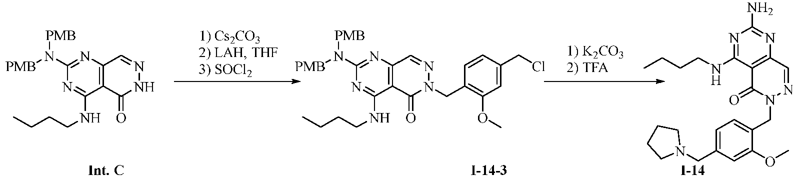

Compound I-14 was synthesized with reference to Compound I-1 by using Intermediate Int. C instead of Intermediate Int. A and Methyl 4-(bromomethyl)-3-methoxybenzoate instead of methyl 4-(bromomethyl) benzoate to obtain a white solid Compound I-14. MS: 438.2 [M+H]⁺.¹H NMR (400 MHz, DMSO-*d₆*) δ 9.00 (t, J = 5.7 Hz, 1H), 7.83 (s, 1H), 7.04 (br, 2H), 6.94 (s, 1H), 6.82-6.73 (m, 2H), 5.13 (s, 2H), 3.79 (s, 3H), 3.54 (s, 2H), 3.43 (t, J = 7.1 Hz, 2H), 2.46-2.38 (m, 4H), 1.71-1.65 (m, 4H), 1.58-1.50 (m, 2H), 1.39-1.28 (m, 2H), 0.90 (t, J = 7.3 Hz, 3H)

### Compound I-15

### 2-amino-4-(butylamino)-6-(2-fluoro-4-(pyrrolidin-1-ylmethyl)benzyl)pyri mido[4,5-d]pyridazin-5(6H)-one

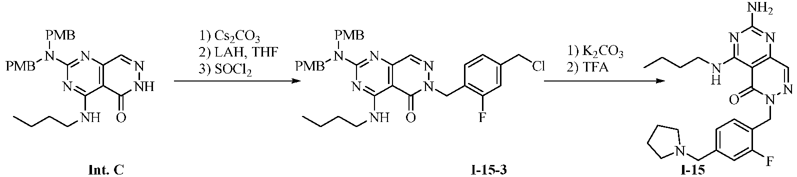

Compound I-15 was synthesized with reference to Compound I-14 by using methyl 4-(bromomethyl)-3-fluoro benzoate instead of methyl 4-(bromomethyl)-3-methoxy benzoate to obtain white solid compound I-15. MS: 426.2 [M+H]⁺.¹H NMR (400 MHz, DMSO-*d₆*) δ 8.95 (t, J = 5.6 Hz, 1H), 7.83 (s, 1H), 7.20-6.98 (m, 5H), 5.21 (s, 2H), 3.54 (s, 2H), 3.45-3.39 (m, 2H), 2.43-2.36 (m, 4H), 1.72-1.62 (m, 4H), 1.58-1.49 (m, 2H), 1.39-1.28 (m, 2H), 0.90 (t, J = 7.3 Hz, 3H).

### Compound I-16

### 2-amino-4-(butylamino)-6-((2-methoxy-6-(pyrrolidin-1-ylmethyl)pyridin-3 -yl)methyl)pyrimido[4,5-d]pyridazin-5(6H)-one

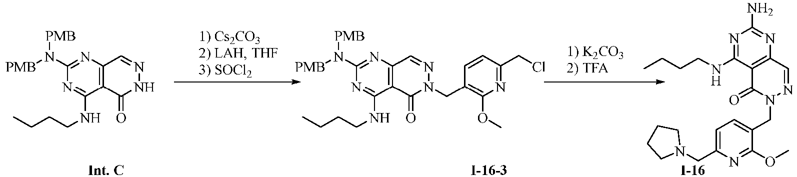

Compound I-16 was synthesized with reference to Compound I-14 by using methyl 5-(bromomethyl) -6-methoxypyridinate formate instead of methyl 4-(bromomethyl)-3- methoxybenzoate to obtain white solid compound I-16. MS: 439.2 [M+H]⁺.¹H NMR (400 MHz, DMSO-*d₆*) δ 8.96 (t, J = 5.7 Hz, 1H), 7.84 (s, 1H), 7.26 (d, J = 7.5 Hz, 1H), 7.06 (br, 2H), 6.93 (d, J = 7.5 Hz, 1H), 5.10 (s, 2H), 3.87 (s, 3H), 3.62 (s, 2H), 3.46-3.37 (m, 1H), 2.54-2.50 (m, 4H), 1.72-1.66 (m, 4H), 1.57-1.49 (m, 2H), 1.39-1.27 (m, 2H), 0.89 (t, J = 7.3 Hz, 3H)

### Compound I-17

### 2-amino-4-(butylamino)-6-(3-methoxy-4-(pyrrolidin-1-ylmethyl)benzyl)py rimido[4,5-d]pyridazin-5(6H)-one

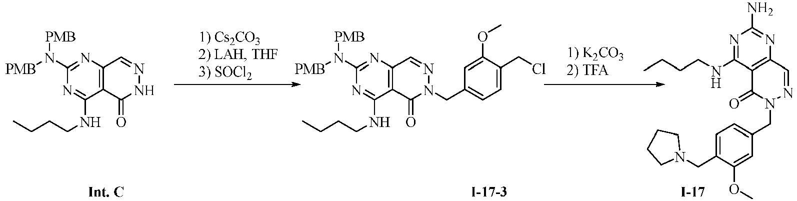

Compound I-17 was synthesized with reference to Compound I-14 by using methyl 4-(bromomethyl)-2- methoxybenzoate instead of methyl 4-(bromomethyl)-3- methoxybenzoate to obtain white solid compound I-17. MS: 438.2 [M+H]⁺.¹H NMR (400 MHz, DMSO-*d₆*) δ 9.00 (t, J = 5.7 Hz, 1H), 7.84 (s, 1H), 7.23 (d, J = 7.7 Hz, 1H), 7.05 (br, 1H), 6.92 (d, J = 1.6 Hz, 1H), 6.77 (dd, J = 7.6, 1.5 Hz, 1H), 5.16 (s, 2H), 3.74 (s, 3H), 3.56 (s, 2H), 3.46-3.39 (m, 2H), 2.48-2.43 (m, 4H), 1.71-1.63 (m, 4H), 1.58-1.50 (m, 2H), 1.39-1.28 (m, 2H), 0.90 (t, J = 7.3 Hz, 3H)

### Compound I-18

### 2-amino-4-(butylamino)-6-(3-fluoro-4-(pyrrolidin-1-ylmethyl)benzyl)pyri mido[4,5-d]pyridazin-5(6H)-one

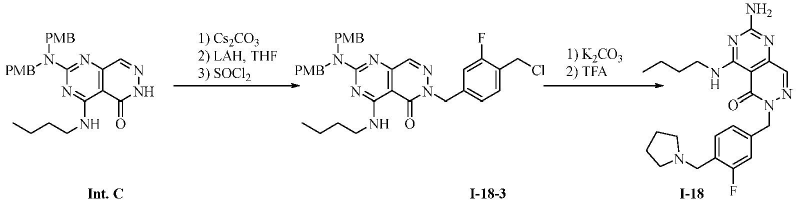

Compound I-18 was synthesized with reference to Compound I-14 by using methyl 4-(bromomethyl)-2-fluoro benzoate instead of methyl 4-(bromomethyl)-3-methoxy benzoate to obtain white solid compound I-18. MS: 426.2 [M+H]⁺.¹H NMR (400 MHz, DMSO-*d₆*) δ 8.96 (t, J = 5.7 Hz, 1H), 7.84 (s, 1H), 7.35 (t, J = 7.9 Hz, 1H), 7.14-6.96 (m, 4H), 5.17 (s, 2H), 3.55 (s, 2H), 3.42 (q, J = 6.6 Hz, 2H), 2.43-2.37 (m, 4H), 1.67-1.63 (m, 4H), 1.57-1.50 (m, 2H), 1.39-1.28 (m, 2H), 0.90 (t, J = 7.3 Hz, 3H).

### Compound I-19

### 2-amino-4-(butylamino)-6-(4-(2-(methylamino)ethoxy)benzyl)pyrimido[4, 5-d]pyridazin-5(6H)-one

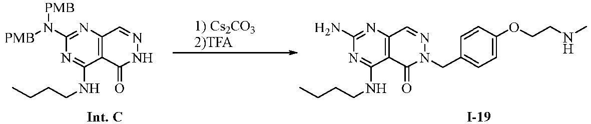

Compound I-19 was synthesized with reference to Compound I-14 by using 2-(4-(bromomethyl)phenoxy)-N-methylethan-1-amine instead of methyl 4-(bromomethyl)-3-methoxy benzoate to obtain white solid compound I-19. MS: 398.2 [M+H]⁺.¹H NMR (400 MHz, DMSO-*d₆*) δ 9.00 (t, J = 5.7 Hz, 1H), 8.27 (d, J = 1.1 Hz, 1H), 7.81 (s, 1H), 7.28-7.18 (m, 2H), 7.03 (br, 2H), 6.94-6.86 (m, 2H), 5.11 (s, 2H), 4.06 (t, J = 5.4 Hz, 2H), 3.45-3.39 (m, 2H), 3.01 (t, J = 5.4 Hz, 2H), 2.43 (s, 3H), 1.58-1.50 (m, 2H), 1.39-1.29 (m, 2H), 0.90 (t, J = 7.4 Hz, 3H).

### Compound I-20

### 2-amino-4-(butylamino)-6-(5-(pyrrolidin-1-yl)pentyl)pyrimido[4,5-d]pyrid azin-5(6H)-one

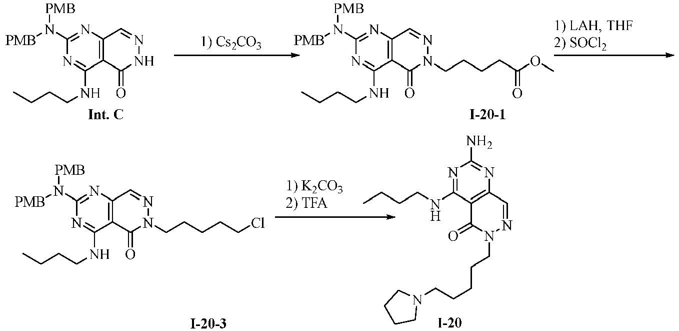

Compound I-20 was synthesized with reference to Compound I-14 by using methyl 5-bromovalerate instead of methyl 4-(bromomethyl)-3-methoxy benzoate to obtain white solid compound I-20. MS: 374.2 [M+H]⁺.¹H NMR (400 MHz, DMSO-*d₆*) δ 9.48 (s, 1H), 9.28 (s, 1H), 7.90 (s, 1H), 7.41 (br, 2H), 4.04 (t, J = 7.0 Hz, 2H), 3.53-3.42 (m, 4H), 3.11-3.05 (m, 2H), 2.99-2.90 (m, 2H), 2.01-1.94 (m, 2H), 1.87-1.80 (m, 2H), 1.75-1.68 (m, 2H), 1.65-1.52 (m, 4H), 1.39-1.26 (m, 4H), 0.91 (t, J = 7.3 Hz, 3H)

### Compound I-21

### 2-amino-4-(butylamino)-6-(4-((2-hydroxyethyl)(propyl)amino)methyl)ben zyl)pyrimido[4,5-d]pyridazin-5(6H)-one

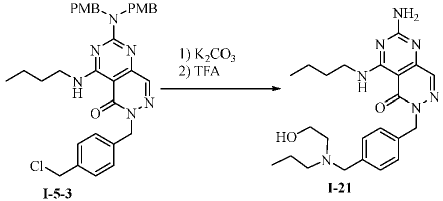

Compound I-21 was synthesized with reference to Compound I-5 by using 2-(propylamino)ethan-1-ol instead of pyrrolidine to obtain white solid compound I-21. MS: 440.2 [M+H]⁺.¹H NMR (400 MHz, DMSO-*d₆*) δ 8.99 (t, J = 5.6 Hz, 1H), 7.83 (s, 1H), 7.29-7.17 (m, 4H), 7.03 (br, 2H), 5.16 (s, 2H), 3.52 (s, 2H), 3.45-3.39 (m, 4H), 2.44 (t, J = 6.7 Hz, 2H), 2.37-2.32 (m, 2H), 1.57-1.50 (m, 2H), 1.44-1.27 (m, 4H), 0.90 (t, J = 7.3 Hz, 3H), 0.79 (t, J = 7.3 Hz, 3H)

### Compound I-22

### 2-amino-4-(butylamino)-6-(5-oxo-5-(piperazin-1-yl)pentyl)pyrimido[4,5-d] pyridazin-5(6H)-one

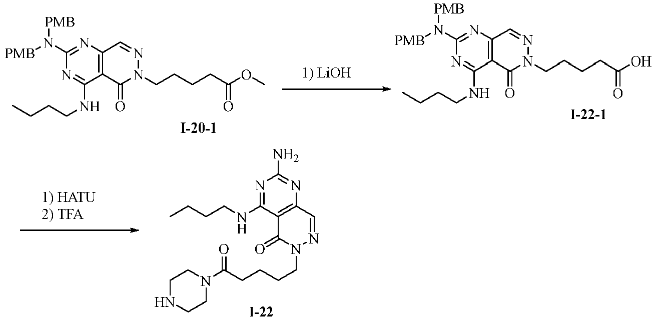

Compound I-22 was synthesized with reference to Compound I-2 by using Intermediate I-20-1 instead of Intermediate I-2-1 to obtain white solid compound I-22. MS: 403.2 [M+H]⁺.¹H NMR (400 MHz, DMSO-*d₆*) δ 9.06 (t, J = 5.6 Hz, 1H), 8.21 (d, J = 1.3 Hz, 1H), 7.80 (s, 1H), 7.00 (br, 2H), 4.00 (t, J = 7.0 Hz, 2H), 3.46-3.37 (m, 6H), 2.79-2.68 (m, 4H), 2.32 (t, J = 7.4 Hz, 2H), 1.73-1.65 (m, 2H), 1.59-1.43 (m, 4H), 1.40-1.30 (m, 2H), 0.91 (t, J = 7.3 Hz, 3H).

### Compound I-23

### 2-amino-6-(4-((bis(2-hydroxyethyl)amino)methyl)benzyl)-4-(butylamino)p yrimido[4,5-d]pyridazin-5(6H)-one

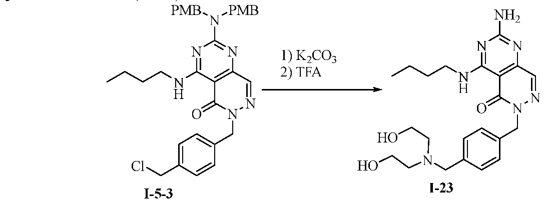

Compound I-23 was synthesized with reference to Compound I-5 by using dihydroxyethylamine instead of pyrrolidine to obtain white solid compound I-23. MS: 442.2 [M+H]⁺.¹H NMR (400 MHz, DMSO-*d₆*) δ 8.99 (t, J = 5.7 Hz, 1H), 7.83 (s, 1H), 7.31-7.18 (m, 4H), 7.03 (br, 2H), 5.16 (s, 2H), 4.33 (br, 2H), 3.58 (s, 2H), 3.45-3.38 (m, 6H), 2.52-2.46 (m, 4H), 1.57-1.50 (m, 2H), 1.40-1.27 (m, 2H), 0.90 (t, J = 7.3 Hz, 3H).

### Compound I-24

### (R)-2-amino-6-(2-methoxy-4-(piperazine-1-carbonyl)benzyl)-4-(pentan-2-y lamino)pyrimido[4,5-d]pyridazin-5(6H)-one

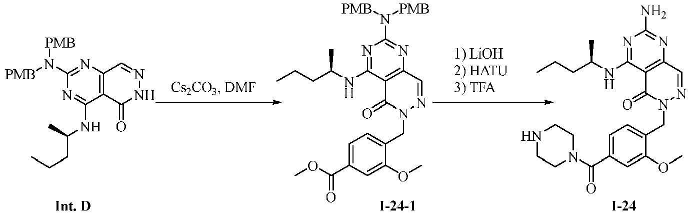

Compound I-24 was synthesized with reference to Compound I-2 by using Intermediate Int. D instead of Intermediate Int. C to obtain white solid compound I-24. MS: 481.2 [M+H]⁺.¹H NMR (400 MHz, DMSO-*d₆*) δ 8.88 (d, J = 8.3 Hz, 1H), 7.85 (s, 1H), 7.12-7.98 (m, 3H), 6.89-6.84 (m, 2H), 5.22-5.09 (m, 2H), 4.20 (p, J = 6.7 Hz, 1H), 3.60-3.48 (m, 2H), 3.90-3.20 (m, 2H), 2.80-2.64 (m, 4H), 1.53-1.43 (m, 2H), 1.37-1.25 (m, 2H), 1.15 (d, J = 6.5 Hz, 3H), 0.87 (t, J = 7.3 Hz, 3H).

### Compound I-25

### (R)-2-amino-6-(2-methoxy-4-(pyrrolidin-1-ylmethyl)benzyl)-4-(pentan-2-y lamino)pyrimido[4,5-d]pyridazin-5(6H)-one

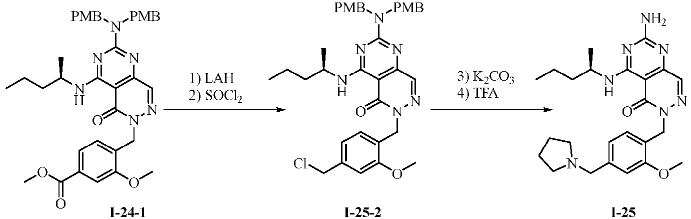

Compound I-25 was synthesized with reference to Compound I-1 by using Intermediate I-24-1 instead of Intermediate I-1-1 to obtain white solid compound I-25. MS: 452.2 [M+H]⁺.¹H NMR (400 MHz, DMSO-*d₆*) δ 8.91 (d, J = 8.3 Hz, 1H), 7.83 (s, 1H), 7.05 (br, 2H), 6.94 (s, 1H), 6.82-6.74 (m, 2H), 5.20-5.06 (m, 2H), 4.20 (p, J = 6.7 Hz, 1H), 3.79 (s, 3H), 3.55 (s, 2H), 2.46-2.40 (m, 4H), 1.71-1.64 (m, 4H), 1.54-1.44 (m, 2H), 1.36-1.26 (m, 2H), 1.15 (d, J = 6.4 Hz, 3H), 0.87 (t, J = 7.3 Hz, 3H).

### Compound I-26

### (R)-2-amino-6-(4-((2-hydroxyethyl)(propyl)amino)methyl)benzyl)-4-(pent an-2-ylamino)pyrimido[4,5-d]pyridazin-5(6H)-one

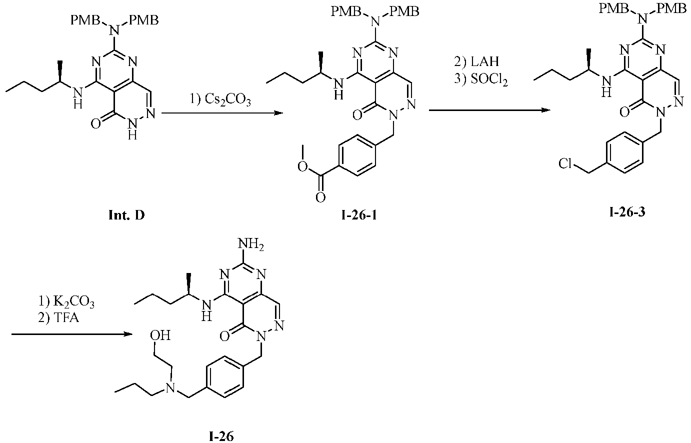

Compound I-26 was synthesized with reference to Compound I-1 by using Intermediate Int. D instead of Intermediate Int. A and 2-(propylamino)ethan-1-ol instead of pyrrolidine to obtain white solid compound I-26. MS: 454.2 [M+H]⁺,¹H NMR (400 MHz, DMSO-*d₆*) δ 8.91 (d, J = 8.3 Hz, 1H), 7.83 (s, 1H), 7.10-6.95 (m, 3H), 6.81-6.73 (m, 2H), 5.20-5.05 (m, 2H), 4.33 (br, 1H), 4.20 (p, J = 6.9 Hz, 1H), 3.79 (s, 3H), 3.53 (s, 2H), 3.44 (t, J = 6.7 Hz, 2H), 2.46 (t, J = 6.6 Hz, 2H), 2.37 (t, J = 7.3 Hz, 2H), 1.53-1.36 (m, 4H), 1.35-1.27 (m, 2H), 1.15 (d, J = 6.6 Hz, 3H), 0.87 (t, J = 7.3 Hz, 3H), 0.81 (t, J = 7.3 Hz, 3H)

### Compound I-27

### (R)-2-amino-6-(4-((bis(2-hydroxyethyl)amino)methyl)benzyl)-4-(pentan-2-ylamino)pyrimido[4,5-d]pyridazin-5(6H)-one

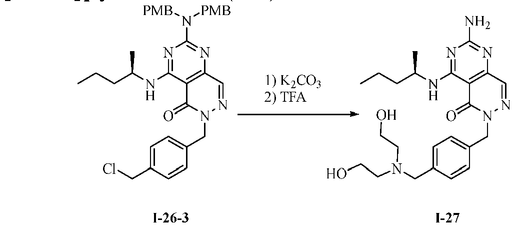

Compound I-27 was synthesized with reference to Compound I-26 by using diethanolamine instead of 2-(propylamino)ethan-1-ol to obtain white solid compound I-27. MS: 456.2 [M+H]⁺.¹H NMR (400 MHz, DMSO-*d₆*) δ 8.91 (d, J = 8.3 Hz, 1H), 7.11-6.95 (m, 3H), 7.01 (s, 3H), 6.83-6.72 (m, 2H), 5.19-5.06 (m, 2H), 4.20 (p, J = 6.9 Hz, 1H), 3.79 (s, 3H), 3.59 (s, 2H), 3.43 (t, J = 6.3 Hz, 4H), 2.54-2.50 (m, 4H), 1.53-1.42 (m, 2H), 1.37-1.25 (m, 2H), 1.15 (d, J = 6.5 Hz, 3H), 0.87 (t, J = 7.2 Hz, 3H).

### Compound I-28

### 2-amino-4-(butylamino)-8-methyl-6-(4-(piperazine-1-carbonyl)benzyl)pyri mido[4,5-d]pyridazin-5(6H)-one

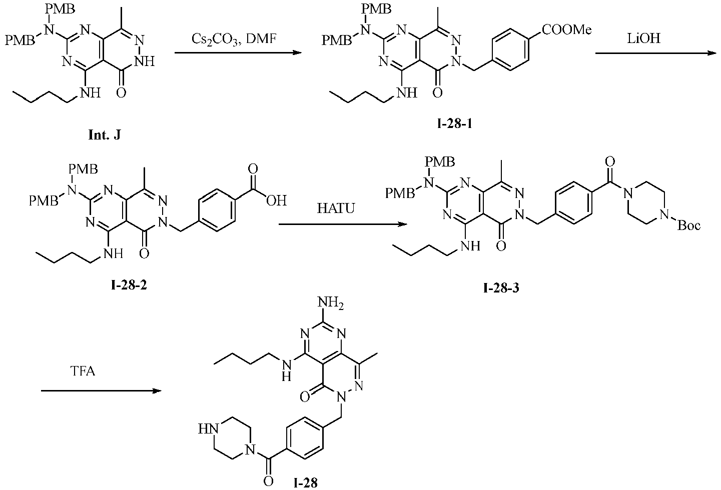

Compound I-28 was synthesized with reference to Compound I-2 by using Intermediate Int. J instead of Intermediate Int. C to obtain white solid compound I-28. MS: 451.2 [M+H]⁺.¹H NMR (400 MHz, DMSO-*d₆*) δ 8.89 (d, J = 8.3 Hz, 1H), 7.36-7.28 (m, 4H), 7.04 (br, 2H), 5.19 (s, 2H), 3.63-3.48 (m, 2H), 3.43 (q, J = 6.6 Hz, 2H), 3.33-3.20 (m, 2H), 2.86 -2.64 (m, 4H), 2.28 (s, 3H), 1.58-1.49 (m, 2H), 1.38-1.28 (m, 2H), 0.89 (t, J = 7.3 Hz, 3H).

### Compound I-29

### 2-amino-4-(butylamino)-8-methyl-6-(4-(pyrrolidin-1-ylmethyl)benzyl)pyri mido[4.5-d]pyridazin-5(6H)-one

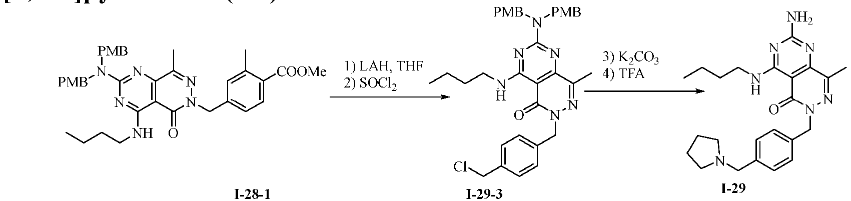

Compound I-29 was synthesized with reference to Compound I-1 by using Intermediate I-28-1 instead of Intermediate I-1-1 to obtain white solid compound I-29. MS: 422.2 [M+H]⁺.¹H NMR (400 MHz, DMSO-*d₆*) δ 9.16 (t, J = 5.6 Hz, 1H), 7.29-7.16 (m, 4H), 7.04 (br, 2H), 5.13 (s, 2H), 3.57 (s, 2H), 3.42 (q, J = 6.6 Hz, 2H), 2.47-2.41 (m, 4H), 2.27 (s, 3H), 1.70-1.63 (m, 4H), 1.57-1.49 (m, 2H), 1.38-1.28 (m, 2H), 0.89 (t, J = 7.3 Hz, 3H).

### Compound I-30

### 2-amino-4-((1-hydroxyhexan-3-yl)amino)-6-(4-(piperazine-1-carbonyl)ben zyl)pyrimido[4,5-d]pyridazin-5(6H)-one

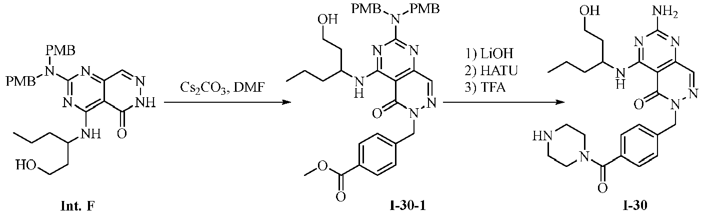

Compound I-30 was synthesized with reference to Compound I-2 by using Intermediate Int. F instead of Intermediate Int. C to obtain white solid compound I-30. MS: 481.2 [M+H]⁺.¹H NMR (400 MHz, DMSO-*d₆*) δ 8.90 (d, J = 8.8 Hz, 1H), 8.22 (s, 1H), 7.84 (s, 1H), 7.39-7.26 (m, 4H), 7.05 (br, 2H), 5.26-5.15 (m, 2H), 4.35-4.25 (m, 1H), 3.60-3.49 (m, 2H), 3.45-3.40 (m, 2H), 3.31-3.19 (m, 2H), 2.85-2.60 (m, 4H), 1.77-1.42 (m, 4H), 1.35-1.21 (m, 2H), 0.86 (t, J = 7.3 Hz, 3H).

### Compound I-31

### 2-amino-4-((1-hydroxyhexan-3-yl)amino)-6-(4-(pyrrolidin-1-ylmethyl)ben zyl)pyrimido[4,5-d]pyridazin-5(6H)-one

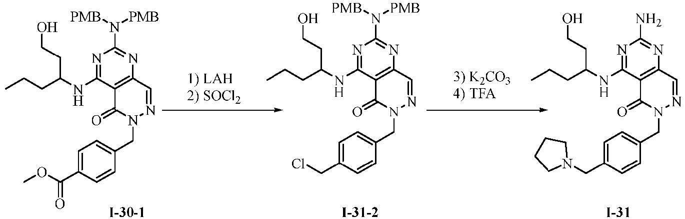

Compound I-31 was synthesized with reference to Compound I-1 by using Intermediate I-30-1 instead of Intermediate I-1-1 to obtain white solid compound I-31. MS: 452.2 [M+H]⁺.¹H NMR (400 MHz, DMSO-*d₆*) δ 8.92 (d, J = 8.8 Hz, 1H), 7.83 (s, 1H), 7.28-7.20 (m, 4H), 7.02 (br, 2H), 5.19-5.11 (m, 2H), 4.35-4.25 (m, 1H), 3.57 (s, 2H), 3.45-3.39 (m, 2H), 2.46-2.40 (M, 4H), 1.76-1.45 (m, 8H), 1.35-2.23 (m, 2H), 0.86 (t, J = 7.3 Hz, 3H).

### Compound I-32

### 2-amino-4-((1-hydroxyhexan-3-yl)amino)-6-(4-(4-methylpiperazine-1-carb onyl)benzyl)pyrimido[4,5-d]pyridazin-5(6H)-one

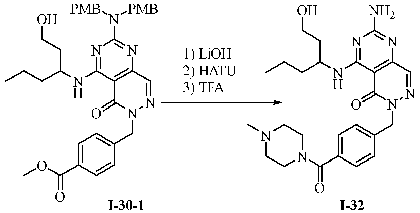

Compound I-32 was synthesized with reference to Compound I-30 by using 1-methylpiperazine instead of tert-butyl piperazine-1-carboxylate to obtain white solid compound I-32. MS: 495.2 [M+H]⁺.¹H NMR (400 MHz, DMSO-*d₆*) δ 8.90 (d, J = 8.8 Hz, 1H), 7.84 (s, 1H), 7.36-7.30 (m, 4H), 7.06 (br, 2H), 5.28-5.15 (m, 2H), 4.35-4.26 (m, 1H), 3.63-3.53 (m, 2H), 3.46-3.40 (m, 2H), 3.32-3.24 (m, 2H), 2.38-2.22 (m, 4H), 2.16 (s, 3H), 1.76-1.43 (m, 4H), 1.35-1.21 (m, 2H), 0.86 (t, J = 7.3 Hz, 3H).

### Compound I-33

### (S)-2-amino-6-(2-methoxy-4-(pyrrolidin-1-ylmethyl)benzyl)-4-(pentan-2-yl amino)pyrimido[4,5-d]pyridazin-5(6H)-one

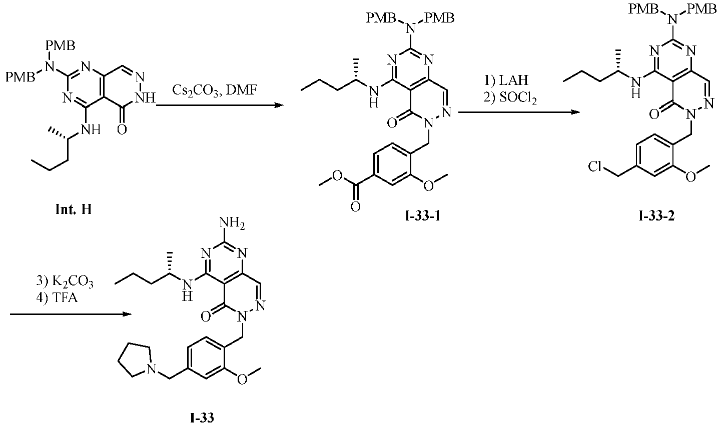

Compound I-33 was synthesized with reference to Compound I-25 by using Intermediate H instead of Intermediate D to obtain white solid compound I-33. MS: 452.2 [M+H]⁺.¹H NMR (400 MHz, DMSO-*d₆*) δ 8.91 (d, J = 8.3 Hz, 1H), 7.83 (s, 1H), 7.05 (br, 2H), 6.94 (s, 1H), 6.82-6.74 (m, 2H), 5.20-5.06 (m, 2H), 4.20 (p, J = 6.7 Hz, 1H), 3.79 (s, 3H), 3.55 (s, 2H), 2.46-2.40 (m, 4H), 1.71-1.64 (m, 4H), 1.54-1.44 (m, 2H), 1.36-1.26 (m, 2H), 1.15 (d, J = 6.4 Hz, 3H), 0.87 (t, J = 7.3 Hz, 3H).

### Compound I-34

### (S)-2-amino-6-(4-((bis(2-hydroxyethyl)amino)methyl)-2-methoxybenzyl)-4 -(pentan-2-ylamino)pyrimido[4,5-d]pyridazin-5(6H)-one

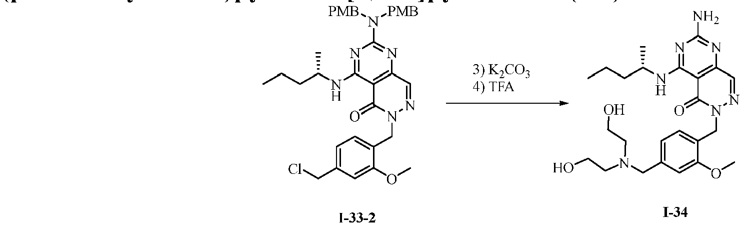

Compound I-34 was synthesized with reference to Compound I-33 by using diethanolamine instead of pyrrolidine to obtain white solid compound I-34. MS: 486.2 [M+H]⁺.¹H NMR (400 MHz, DMSO-*d₆*) δ 8.91 (d, J = 8.3 Hz, 1H), 7.11-6.95 (m, 3H), 7.01 (s, 3H), 6.83-6.71 (m, 2H), 5.20-5.06 (m, 2H), 4.20 (p, J = 6.9 Hz, 1H), 3.79 (s, 3H), 3.59 (s, 2H), 3.43 (t, J = 6.3 Hz, 4H), 2.55-2.50 (m, 4H), 1.53-1.43 (m, 2H), 1.37-1.25 (m, 2H), 1.15 (d, J = 6.5 Hz, 3H), 0.87 (t, J = 7.2 Hz, 3H).

### Compound I-35

### (S)-2-amino-6-(2-methoxy-4-(piperazine-1-carbonyl)benzyl)-4-(pentan-2-y lamino)pyrimido[4,5-d]pyridazin-5(6H)-one

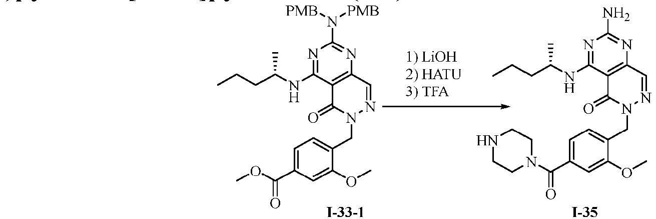

Compound I-35 was synthesized with reference to Compound I-24 by using Intermediate I-33-1 instead of Intermediate I-24-1 to obtain white solid compound I-35. MS: 481.2 [M+H]⁺.¹H NMR (400 MHz, DMSO-*d₆*) δ 9.11 (d, J = 8.3 Hz, 1H), 7.95 (s, 1H), 7.48 (br, 2H) 7.08 (s, 1H), 6.94 (s, 2H), 5.29-5.14 (m, 2H), 4.27-4.20 (m, 1H), 3.85 (s, 3H), 3.65-3.48 (m, 4H), 3.20-3.07 (m, 4H), 1.55-1.48 (m, 2H), 1.38-1.26 (m, 2H), 1.18 (d, J = 6.5 Hz, 3H), 0.88 (t, J = 7.3 Hz, 3H).

### Compound I-36

### (R)-2-amino-6-(4-(4-(4-(2-hydroxyethyl)piperazine-1-carbonyl)-2-methoxy benzyl)-4-(pentan-2-ylamino)pyrimido[4,5-d]pyridazin-5(6H)-one

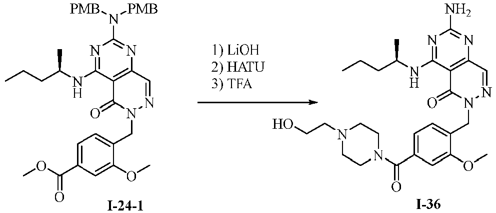

Compound I-36 was synthesized with reference to Compound I-24 by using 2-(piperazin-1-yl)ethan-1-ol instead of tert-butyl piperazine-1-carboxylate to obtain white solid compound I-36. MS: 525.2 [M+H]⁺.¹H NMR (400 MHz, DMSO-*d₆*) δ 9.24 (d, J = 8.3 Hz, 1H), 8.00 (s, 1H), 7.75 (br, 2H), 7.07 (d, J = 1.2 Hz, 1H), 7.00-6.93 (m, 2H), 5.31-5.17 (m, 2H), 4.30-4.15 (m, 4H), 3.75-3.70 (m, 2H), 3.50-3.35 (m, 2H), 3.26-3.07 (m, 4H), 1.59-1.48 (m, 2H), 1.38-1.26 (m, 2H), 1.19 (d, J = 6.4 Hz, 3H), 0.88 (t, J = 7.3 Hz, 3H).

### Compound I-37

### (S)-2-amino-6-(4-(4-(2-hydroxyethyl)piperazine-1-carbonyl)-2-methoxybe nzyl)-4-(pentan-2-ylamino)pyrimido[4,5-d]pyridazin-5(6H)-one

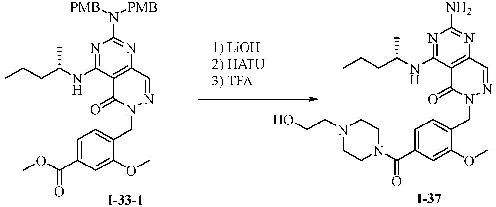

Compound I-37 was synthesized with reference to Compound I-35 by using 2-(piperazin-1-yl)ethan-1-ol instead of tert-butyl piperazine-1-carboxylate to obtain white solid compound I-37. MS: 525.2 [M+H]⁺.¹H NMR (400 MHz, DMSO-*d₆*) δ 9.24 (d, J = 8.3 Hz, 1H), 8.00 (s, 1H), 7.75 (br, 2H), 7.07 (d, J = 1.2 Hz, 1H), 7.00-6.93 (m, 2H), 5.31-5.17 (m, 2H), 4.30-4.15 (m, 4H), 3.75-3.70 (m, 2H), 3.50-3.35 (m, 2H), 3.26-3.07 (m, 4H), 1.59-1.48 (m, 2H), 1.38-1.26 (m, 2H), 1.19 (d, J = 6.4 Hz, 3H), 0.88 (t, J = 7.3 Hz, 3H).

### Compound I-38

### (S)-2-amino-6-(4-((2-hydroxyethyl)(propyl)amino)methyl)-2-methoxybenz yl)-4-(pentan-2-ylamino)pyrimido[4,5-d]pyridazin-5(6H)-one

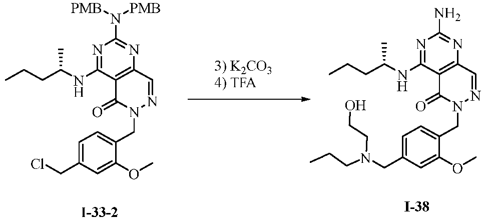

Compound I-38 was synthesized with reference to Compound I-33 by using 2-(propylamino)ethan-1-ol instead of pyrrolidine to obtain white solid compound I-38. MS: 484.2 [M+H]⁺.

### Compound I-39

### (R)-2-amino-6-(4-(2-(methylamino)ethoxy)benzyl)-4-(pentan-2-ylamino)py rimido[4,5-d]pyridazin-5(6H)-one

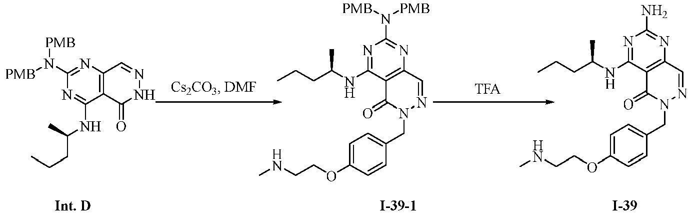

Compound I-39 was synthesized with reference to Compound I-25 by using 2-(4-(bromomethyl)phenoxy)-N-methylethan-1-amine instead of methyl 4-(bromomethyl)-3-methoxy benzoate to obtain white solid compound I-39. MS: 412.2 [M+H]⁺.¹H NMR (400 MHz, DMSO-*d₆*) δ 9.17 (s, 1H), 8.65 (s, 1H), 7.93 (s, 1H), 7.52 (br, 2H), 7.32-7.22 (m, 2H), 7.00-6.90 (m, 2H), 5.24-5.06 (m, 2H), 4.27-4.20 (m, 1H), 4.19-4.15 (m, 2H), 3.36-3.27 (m, 2H), 2.62 (t, J = 5.4 Hz, 3H), 1.57-1.48 (m, 2H), 1.36-1.26 (m, 2H), 1.18 (d, J = 6.5 Hz, 3H), 0.88 (t, J = 7.3 Hz, 3H).

### Compound I-40

### (R)-2-amino-4-(pentan-2-ylamino)-6-(4-(pyrrolidin-1-ylmethyl)benzyl)pyr imido[4,5-d]pyridazin-5(6H)-one

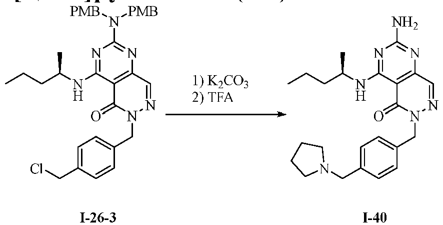

Compound I-40 was synthesized with reference to Compound I-26 by using pyrrolidine instead of 2-(propylamino)ethan-1-ol to obtain white solid compound I-40. MS: 422.2 [M+H]⁺.¹H NMR (400 MHz, DMSO-*d₆*) δ 8.90 (d, J = 8.3 Hz, 1H), 7.83 (s, 1H), 7.28-7.20 (m, 4H), 7.02 (br, 2H), 5.23-5.08 (m, 2H), 4.25-4.15 (m, 1H), 3.56 (s, 2H), 2.47-2.38 (m, 4H), 1.70-1.63 (m, 4H), 1.55-1.42 (m, 2H), 1.37-1.24 (m, 2H), 1.16 (d, J = 6.5 Hz, 3H), 0.87 (t, J = 7.3 Hz, 3H).

### Compound I-41

### (R)-6-(4-((2-oxo-6-azaspiro[3.3]heptan-6-yl)methyl)benzyl)-2-amino-4-(pe ntan-2-ylamino)pyrimido[4,5-d]pyridazin-5(6H)-one

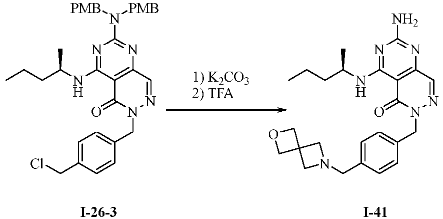

Compound I-41 was synthesized with reference to Compound I-26 by using 2-oxo-6-azaspiro[3.3]heptane instead of 2-(propylamino)ethan-1-ol to obtain white solid compound I-41. MS: 450.2 [M+H]⁺.¹H NMR (400 MHz, DMSO-*d₆*) δ 8.89 (d, J = 8.3 Hz, 1H), 7.83 (s, 1H), 7.23-7.16 (m, 4H), 7.03 (br, 2H), 5.21-5.08 (m, 2H), 4.57 (s, 4H), 4.25-4.15 (m, 1H), 3.44 (s, 2H), 3.25 (s, 4H), 1.55-1.45 (m, 2H), 1.37-1.26 (m, 2H), 1.16 (d, J = 6.5 Hz, 3H), 0.88 (t, J = 7.3 Hz, 3H).

### Compound I-42

### (R)-2-amino-6-(4-((2-hydroxyethyl)amino)methyl)benzyl)-4-(pentan-2-yla mino)pyrimido[4,5-d]pyridazin-5(6H)-one

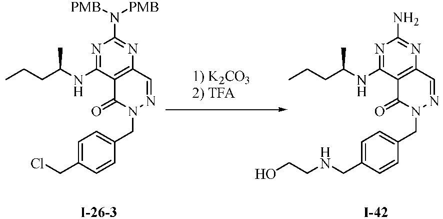

Compound I-42 was synthesized with reference to Compound I-26 by using ethanolamine instead of 2-(propylamino)ethan-1-ol to obtain white solid compound I-42. MS: 412.2 [M+H]⁺.¹H NMR (400 MHz, DMSO-*d₆*) δ 9.06 (s, 1H), 8.83 (s, 1H), 7.92 (s, 1H), 7.46 (d, J = 7.9 Hz, 2H), 7.39 (br, 2H), 7.33 (d, J = 7.9 Hz, 2H), 5.30-5.18 (m 2H), 4.27-4.17 (m, 1H), 4.15-4.08 (m, 2H), 3.65-3.60 (m, 2H), 2.98-2.90 (m, 2H), 1.56-1.45 (m, 2H), 1.35-1.25 (m, 2H), 1.17 (d, J = 6.6 Hz, 3H), 0.88 (t, J = 7.3 Hz, 3H).

### Compound I-43

### (R)-6-(4-((2-oxo-6-azaspiro[3.3]heptan-6-yl)methyl)-2-methoxybenzyl)-2-a mino-4-(pentan-2-ylamino)pyrimido[4,5-d]pyridazin-5(6H)-one

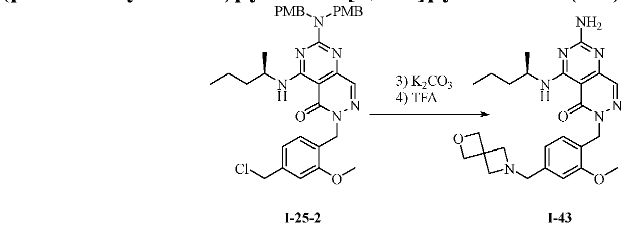

Compound I-43 was synthesized with reference to Compound I-25 by using 2-oxo-6-azaspiro[3.3]heptane instead of pyrrolidine to obtain white solid compound I-43. MS: 480.2 [M+H]⁺.¹H NMR (400 MHz, DMSO-*d₆*) δ 10.19 (s, 1H), 9.16 (d, J = 8.4 Hz, 1H), 7.95 (s, 1H), 7.59 (br, 2H), 7.08 (s, 1H), 6.92 (s, 2H), 5.24 - 5.12 (m, 2H), 4.68 (s, 2H), 4.60 (s, 2H), 4.30-4.15 (m, 7H), 3.83 (s, 3H), 1.55-1.47 (m, 2H), 1.36-1.26 (m, 2H), 1.17 (d, J = 6.5 Hz, 3H), 0.87 (t, J = 7.3 Hz, 3H).

### Compound I-44

### (R)-2-amino-6-(4-((2-hydroxyethyl)amino)methyl)-2-methoxybenzyl)-4-(p entyl-2-amino)pyrimido[4,5-d]pyridazin-5(6H)-one

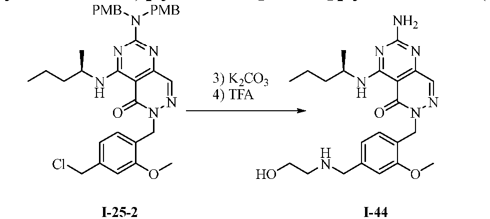

Compound I-44 was synthesized with reference to Compound I-25 by using ethanolamine instead of pyrrolidine to obtain white solid compound I-44. MS: 442.2 [M+H]⁺.¹H NMR (400 MHz, DMSO-*d₆*) δ 9.12 (d, J = 8.3 Hz, 1H), 8.86 (s, 1H), 7.93 (s, 1H), 7.50 (br, 2H), 7.21 (s, 1H), 6.98 (d, J = 7.8 Hz, 1H), 6.92 (d, J = 7.8 Hz, 1H), 5.23 - 5.11 (m, 2H), 4.26-1.18 (m, 1H), 4.15-4.09 (m, 2H), 3.83 (s, 3H), 2.98-2.90 (m, 2H), 1.55-1.45 (m, 2H), 1.35-1.28 (m, 2H), 1.17 (d, J = 6.5 Hz, 3H), 0.87 (t, J = 7.3 Hz, 3H).

### Compound I-45

### 2-amino-4-(butylamino)-6-((5-methoxy-1,2,3,4-tetrahydroisoquinolin-7-yl) methyl)pyrimido[4,5-d]pyridazin-5(6H)-one

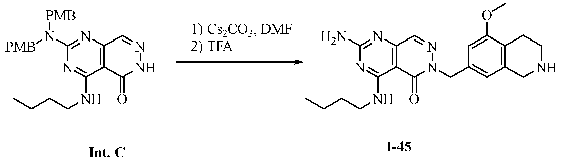

Compound I-45 was synthesized with reference to Compound I-2 by using tert-butyl 7-(bromomethyl)-5-methoxy-3,4-dihydroisoquinoline-2(1H)-carboxylate instead of methyl 4-(bromomethyl) benzoate to obtain white solid compound I-45. MS: 410.2 [M+H]⁺.¹H NMR (400 MHz, DMSO-*d₆*) δ 9.14 (s, 1H), 8.91 (s, 2H), 7.90 (s, 1H), 7.32 (br, 2H), 6.89 (s, 1H), 6.64 (s, 1H), 5.18 (s, 2H), 4.21-4.16 (m, 2H), 3.78 (s, 3H), 3.49-3.41 (m, 2H), 3.38-3.30 (m, 2H), 2.77-2.72 (m, 2H), 1.61-1.52 (m, 2H), 1.38-1.31 (m, 2H), 0.90 (t, J = 7.3 Hz, 3H).

### Compound I-46

### (R)-2-amino-6-(2-methoxy-4-(2-(methylamino)ethoxy)benzyl)-4-(pentan-2-ylamino)pyrimido[4,5-d]pyridazin-5(6H)-one

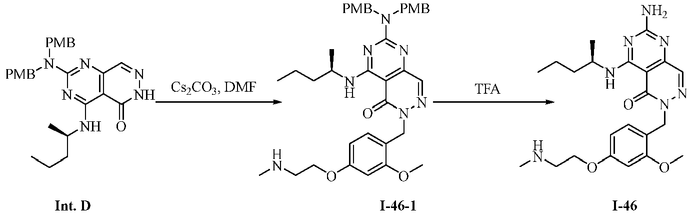

Compound I-46 was synthesized with reference to Compound I-25 by using 2-(4-(bromomethyl)-3-methoxyphenoxy)-N-methylethan-1-amine instead of methyl 4-(bromomethyl)-3-methoxy benzoate to obtain white solid compound I-46. MS: 442.2 [M+H]⁺.¹H NMR (400 MHz, DMSO-*d₆*) δ 8.93 (d, J = 8.3 Hz, 1H), 7.81 (s, 1H), 7.01 (br, 2H), 6.83 (d, J = 8.4 Hz, 1H), 6.59 (d, J = 2.4 Hz, 1H), 6.47 (dd, J = 8.4, 2.4 Hz, 1H), 5.15 -5.01 (m, 2H), 4.25-4.17 (m, 1H), 4.10-4.05 (t, J = 5.3 Hz, 2H), 3.02 (s, 2H), 2.44 (s, 3H), 1.55-1.44 (m, 2H), 1.37-1.27 (m, 2H), 1.16 (d, J = 6.5 Hz, 3H), 0.88 (t, J = 7.3 Hz, 3H).

### Compound I-47

### (R)-2-amino-6-((6-(2-(methylamino)ethoxy)pyridin-3-yl)methyl)-4-(pentan -2-ylamino)pyrimido[4,5-d]pyridazin-5(6H)-one

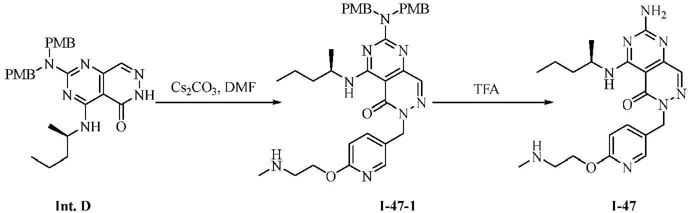

Compound I-47 was synthesized with reference to Compound I-25 by using 2-((5-(bromomethyl)pyridin-2-yl)oxy)-N-methylethan-1-amine instead of methyl 4-(bromomethyl)-3-methoxy benzoate to obtain white solid compound I-47. MS: 413.2 [M+H]⁺.¹H NMR (400 MHz, DMSO-*d₆*) δ 8.88 (d, J = 8.3 Hz, 1H), 8.28 (s, 1H), 8.14 (d, J = 2.5 Hz, 1H), 7.82 (s, 1H), 7.66 (dd, J = 8.6, 2.5 Hz, 1H), 7.03 (br, 2H), 6.79 (d, J = 8.6 Hz, 1H), 5.21-5.06 (m, 2H), 4.36-4.30 (m, 2H), 4.25-4.17 (m, 1H), 3.00-2.94 (m, 2H), 2.40 (s, 3H), 1.57-1.45 (m, 2H), 1.39-1.27 (m, 2H), 1.16 (d, J = 6.5 Hz, 3H), 0.88 (t, J = 7.3 Hz, 3H).

### Compound I-48

### (R)-2-amino-6-(4-((4-methylpiperazin-1-yl)methyl)benzyl)-4-(pentan-2-yla mino)pyrimido[4,5-d]pyridazin-5(6H)-one

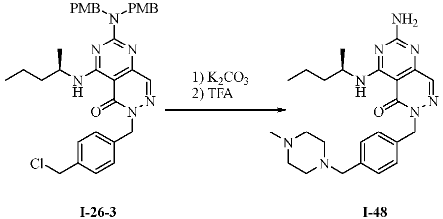

Compound I-48 was synthesized with reference to Compound I-26 by using 4-methylpiperazine instead of 2-(propylamino)ethan-1-ol to obtain white solid compound I-48. MS: 451.2 [M+H]⁺.¹H NMR (400 MHz, DMSO-*d₆*) δ 8.89 (d, J = 8.3 Hz, 1H),7.84 (s, 1H), 7.30-7.22 (m, 4H), 7.03 (br, 2H), 5.26-5.09 (m, 2H), 4.26-4.17 (m, 1H), 3.54-3.49 (m, 2H), 3.36-3.29 (m, 4H), 2.75-2.70 (m, 2H), 2.53 (s, 3H), 1.55-1.44 (m, 2H), 1.36-1.26 (m, 2H), 1.16 (d, J = 6.5 Hz, 3H), 0.88 (t, J = 7.3 Hz, 3H).

### Compound I-49

### (R)-2-amino-6-(4-((2-hydroxyethyl)(methyl)amino)methyl)benzyl)-4-(pent an-2-ylamino)pyrimido[4,5-d]pyridazin-5(6H)-one

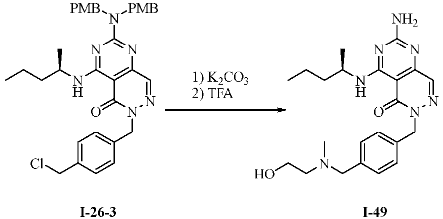

Compound I-49 was synthesized with reference to Compound I-26 by using 2-(methylamino)ethan-1-ol instead of 2-(propylamino)ethan-1-ol to obtain white solid compound I-49. MS: 426.2 [M+H]⁺.¹H NMR (400 MHz, DMSO-*d₆*) δ 8.89 (d, J = 8.3 Hz, 1H), 7.84 (s, 1H), 7.33 (d, J = 7.9 Hz, 2H), 7.27 (d, J = 7.9 Hz, 2H), 7.03 (br, 2H), 5.26-5.07 (m, 2H), 4.25-4.17 (m, 1H), 3.75 (s, 2H), 3.56 (t, J = 6.0 Hz, 2H), 2.68-2.60 (m, 2H), 2.33 (s, 3H), 1.56-1.45 (m, 2H), 1.36-1.25 (m, 2H), 1.16 (d, J = 6.5 Hz, 3H), 0.88 (t, J = 7.3 Hz, 3H).

### Compound I-50

### (R)-2-amino-4-(pentan-2-ylamino)-6-(4-(piperazin-1-ylmethyl)benzyl)pyri mido[4.5-d]pyridazin-5(6H)-one

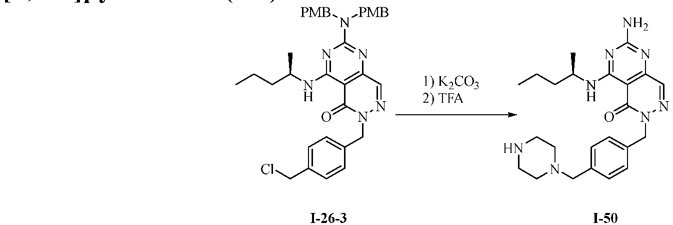

Compound I-50 was synthesized with reference to Compound I-26 by using tert-butyl piperazine-1-carboxyliate instead of 2-(propylamino)ethan-1-ol to obtain white solid compound I-50. MS: 437.2 [M+H]⁺.¹H NMR (400 MHz, DMSO-*d₆*) δ 8.89 (d, J = 8.3 Hz, 1H), 7.83 (s, 1H), 7.27-7.21 (m, 4H),7.05 (br, 2H), 5.23-5.08 (m, 2H), 4.21 (p, J = 6.8 Hz, 1H), 3.45 (s, 2H), 2.93-2.87 (m, 4H), 2.44-2.38 (m, 4H), 1.55-1.44 (m, 2H), 1.37-1.25 (m, 2H), 1.16 (d, J = 6.5 Hz, 3H), 0.88 (t, J = 7.3 Hz, 3H).

### Compound I-51

### 2-amino-4-(((R)-pentan-2-yl)amino)-6-(4-((R)-1-(propylamino)ethyl)benzy 1)pyrimido[4,5-d]pyridazine-5(6H)-one

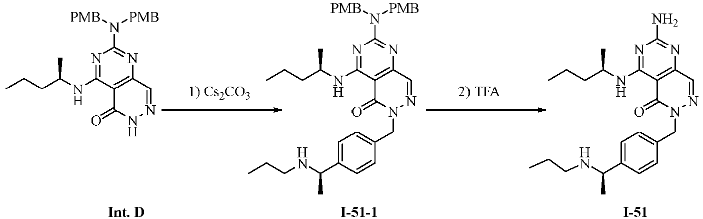

Compound I-51 was synthesized with reference to Compound I-26 by using tert-butyl (R)-(1-(4-(chloromethyl)phenyl)ethyl)(propyl)carbamate instead of methyl 4-(bromomethyl) benzoate to obtain white solid compound I-51. MS: 424.2 [M+H]⁺.¹H NMR (400 MHz, DMSO-*d₆*) δ 8.89 (d, J = 8.3 Hz, 1H), 7.83 (s, 1H), 7.35 (d, J = 8.1 Hz, 2H), 7.27 (d, J = 7.9 Hz, 2H), 7.05 (br, 2H), 5.25-5.08 (m, 2H), 4.21 (p, J = 6.7 Hz, 1H), 3.96-3.89 (m, 1H), 2.53-2.46 (m, 1H), 2.38-2.29 (m, 1H), 1.54-1.38 (m, 4H), 1.35-1.27 (m, 5H), 1.16 (d, J = 6.5 Hz, 3H), 0.88 (t, J = 7.3 Hz, 3H), 0.81 (t, J = 7.4 Hz, 3H).

### Compound I-52

### 2-amino-4-(((R)-pentan-2-yl)amino)-6-(4-((S)-1-(propylamino)ethyl)benzy 1)pyrimido[4,5-d]pyridazine-5(6H)-one

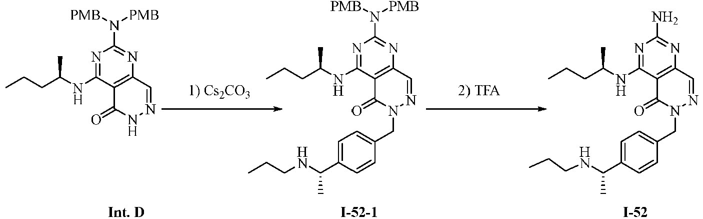

Compound I-52 was synthesized with reference to Compound I-26 by using tert-butyl (S)-(1-(4-(chloromethyl)phenyl)ethyl)(propyl)carbamate instead of methyl 4-(bromomethyl) benzoate to obtain white solid compound I-52. MS: 424.2 [M+H]⁺.¹H NMR (400 MHz, DMSO-*d₆*) δ 8.88 (d, J = 8.3 Hz, 1H), 7.84 (s, 1H), 7.42 (d, J = 8.1 Hz, 2H), 7.32 (d, J = 8.0 Hz, 2H), 7.04 (br, 2H), 5.27-5.11 (m, 2H), 4.21 (t, J = 7.0 Hz, 2H), 3.40-3.22 (m, 2H), 2.73-2.65 (m, 1H), 2.49-2.43 (m, 1H), 1.58-1.42 (m, 7H), 1.36-1.25 (m, 2H), 1.16 (d, J = 6.5 Hz, 3H), 0.88 (t, J = 7.3 Hz, 3H), 0.83 (t, J = 7.4 Hz, 3H).

### Compound I-53

### (R)-2-amino-6-((2-methoxy-6-(2-(methylamino)ethoxy)pyridin-3-yl)methyl )-4-(pentan-2-ylamino)pyrimido[4,5-d]pyridazin-5(6H)-one

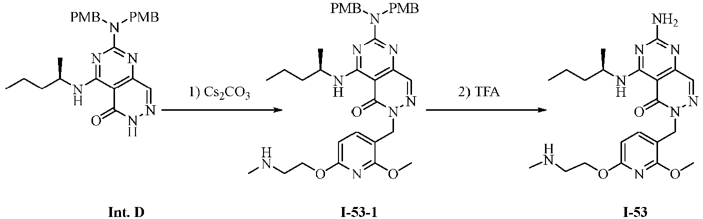

Compound I-53 was synthesized with reference to Compound I-52 by using 2-((5-(bromomethyl)-6-methoxypyridin-2-yl)oxy)-N-methylethan-1-amine instead of tert-butyl (S)-(1-(4-(chloromethyl)phenyl)ethyl)(propyl)carbamate to obtain white solid compound I-53. MS: 443.2 [M+H]⁺.¹H NMR (400 MHz, DMSO-*d₆*) δ 8.89 (d, J = 8.3 Hz, 1H), 7.80 (s, 1H), 7.34 (d, J = 8.1 Hz, 1H), 7.02 (br, 2H), 6.31 (d, J = 8.1 Hz, 1H), 5.12-4.98 (m, 2H), 4.30-4.17 (m, 3H), 3.86 (s, 3H), 2.87-2.78 (m, 2H), 2.33 (s, 3H), 1.53-1.45 (m, 2H), 1.37-1.27 (m, 2H), 1.16 (d, J = 6.5 Hz, 3H), 0.88 (t, J = 7.3 Hz, 3H).

### Compound I-54

### Butyl (R)-4-(4-((2-amino-5-oxo-4-(pentan-2-ylamino)pyrimido[4,5-d]pyridazin-6(5H) -yl)methyl)benzyl)piperazine-1-carboxylate

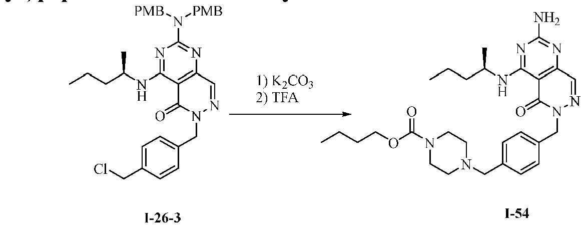

Compound I-54 was synthesized with reference to Compound I-26 by using butyl piperazine-1-carboxylate instead of 2-(propylamino)ethan-1-ol to obtain white solid compound I-54. MS: 537.2 [M+H]⁺.¹H NMR (400 MHz, DMSO-*d₆*) δ 8.90 (d, J = 8.3 Hz, 1H), 7.83 (s, 1H), 7.27-7.21 (m, 4H), 7.03 (br, 2H), 5.23-5.06 (m, 2H), 4.25-4.16 (m, 1H), 3.96 (t, J = 6.5 Hz, 2H), 3.43 (s, 2H), 3.35-3.32 (m, 4H), 2.30-2.25 (m, 4H), 1.55-1.45 (m, 4H), 1.35-1.25 (m, 4H), 1.16 (d, J = 6.5 Hz, 3H), 0.90-1.83 (m, 6H).

### Compound I-55

### (R)-2-amino-4-(pentan-2-ylamino)-6-(4-(piperidin-4-yl)benzyl)pyrimido[4, 5-d]pyridazin-5(6H)-one

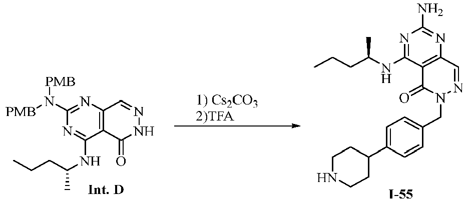

Compound I-55 was synthesized with reference to Compound I-26 by using tert-butyl 4-(4-(bromomethyl)phenyl)piperidine-1-carboxylate instead of methyl 4-(bromomethyl) benzoate to obtain white solid compound I-55. MS: 422.2 [M+H]⁺.¹H NMR (400 MHz, DMSO-*d₆*) δ 8.90 (d, J = 8.3 Hz, 1H), 7.83 (d, J = 2.0 Hz, 1H), 7.42-7.14 (m, 4H), 7.04 (br, 2H), 5.24-5.03 (m, 2H), 4.21 (p, J = 6.8 Hz, 1H), 3.38-3.33 (m, 1H), 3.10-3.03 (m, 1H), 2.93-2.87 (m, 1H), 2.68-2.56 (m, 1H), 2.34-2.28 (m, 1H), 1.88-1.85 (m, 3H), 1.71-1.45 (m, 3H), 1.36-1.24 (m, 2H), 1.16 (d, J = 6.5 Hz, 3H), 0.88 (t, J = 7.3 Hz, 3H).

### Compound I-56

### (R)-2-amino-4-(pentan-2-ylamino)-6-(4-(piperazine-1-carbonyl)benzyl)pyr imido[4,5-d]pyridazin-5(6H)-one

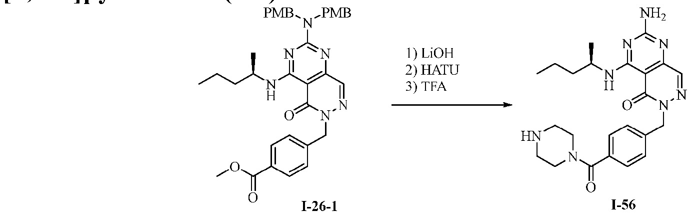

Compound I-56 was synthesized with reference to Compound 1-24 by using Intermediate I- 26-1 instead of Intermediate I- 24-1 to obtain white solid compound I-56. MS: 451.2 [M+H]⁺.¹H NMR (400 MHz, DMSO-*d₆*) δ 9.07 (d, J = 8.1 Hz, 1H), 8.84 (br, 2H), 7.94 (s, 1H), 7.46-7.33 (m, 6H), 5.34-5.18 (m, 2H), 4.23 (p, J = 7.0 Hz, 1H), 3.75-3.50 (m, 4H), 3.19-3.06 (m, 4H), 1.56-1.48 (m, 2H), 1.37-1.27 (m, 2H), 1.18 (d, J = 6.5 Hz, 3H), 0.88 (t, J = 7.3 Hz, 3H).

### Compound I-57

### (R)-2-amino-6-(4-(1-methylpiperidin-4-yl)benzyl)-4-(pentan-2-ylamino)py rimido[4,5-d]pyridazin-5(6H)-one

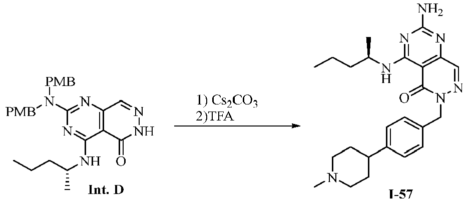

Compound I-57 was synthesized with reference to Compound I-26 by using 4-(4-(bromomethyl)phenyl)-1-methylpiperidine instead of methyl 4-(bromomethyl) benzoate to obtain white solid compound I-57. MS: 436.2 [M+H]⁺.¹H NMR (400 MHz, DMSO-*d₆*) δ 8.90 (d, J = 8.3 Hz, 1H), 7.83 (d, J = 2.4 Hz, 1H), 7.25-7.17 (m, 4H), 7.03 (br, 2H), 5.21-5.03 (m, 2H), 4.20 (p, J = 6.8 Hz, 1H), 3.00-2.98 (m, 2H), 2.58-2.52 (m, 1H), 2.45-2.38 (m, 1H), 2.24 (d, J = 21.7 Hz, 3H), 2.05-1.95 (m, 1H), 1.72-1.57 (m, 3H), 1.54-1.44 (m, 2H), 1.37-1.26 (m, 2H), 1.16 (d, J = 6.5 Hz, 3H), 0.88 (t, J = 7.3 Hz, 3H).

### Compound I-58

### (S)-2-amino-4-((1-hydroxypentan-2-yl)amino)-6-(4-(piperazine-1-carbonyl )benzyl)pyrimido[4,5-d]pyridazin-5(6H)-one

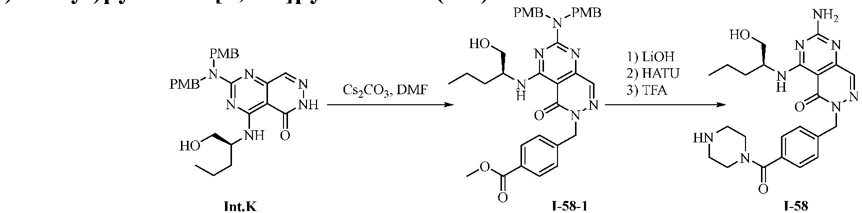

Compound I-58 was synthesized with reference to Compound I-2 by using Intermediate Int. K instead of Intermediate Int. C to obtain white solid compound I-58. MS: 467.2 [M+H]⁺.¹H NMR (400 MHz, DMSO-*d₆*) δ 9.00 (d, J = 8.7 Hz, 1H), 8.21 (s, 1H), 7.84 (s, 1H), 7.40-7.29 (m, 4H), 7.04 (br, 2H), 5.30-5.12 (m, 2H), 4.23-4.13 (m, 1H), 3.65-3.53 (m, 2H), 3.50-3.42 (m, 2H),3.35-3.20 (m, 2H), 2.84-2.66 (m, 4H), 1.63-1.53 (m, 1H), 1.52-1.43(m, 1H), 1.35-1.24 (m, 2H), 0.88 (t, J = 7.3 Hz, 3H).

### Compound I-59

### (S)-2-amino-4-((1-hydroxypentan-2-yl)amino)-6-(4-(pyrrolidin-1-ylmethyl )benzyl)pyrimido[4,5-d]pyridazin-5(6H)-one

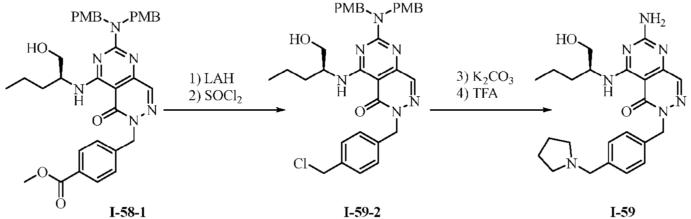

Compound I-59 was synthesized with reference to Compound I-1 by using Intermediate I-58-1 instead of Intermediate I-1-1 to obtain white solid compound I-59. MS: 438.2 [M+H]⁺.¹H NMR (400 MHz, DMSO-*d₆*) δ 9.02 (d, J = 8.7 Hz, 1H), 8.15 (s, 1H), 7.83 (s, 1H), 7.32-7.20 (m, 4H), 7.00 (br, 2H), 5.25-5.06 (m, 2H), 4.84 (br, 1H), 4.23-4.14 (m, 1H), 3.65 (s, 2H), 3.51-3.42 (m, 2H), 2.55-2.50 (m, 4H), 1.72-1.66 (m, 4H), 1.63-1.53 (m, 1H), 1.53-1.41 (m, 1H), 1.36-1.24 (m, 2H), 0.88 (t, J = 7.3 Hz, 3H).

### Compound I-60

### (R)-2-amino-6-(4-(4-methylpiperazine-1-carbonyl)benzyl)-4-(pentan-2-yla mino)pyrimido[4,5-d]pyridazin-5(6H)-one

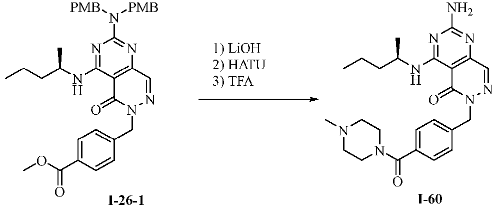

Compound I-60 was synthesized with reference to Compound 1-24 by using Intermediate I-26-1 instead of Intermediate 1-24-1 and 1-methylpiperazine instead of tert-butyl piperazine-1-carboxylateto obtain white solid compound I-60. MS: 465.2 [M+H]⁺.¹H NMR (400 MHz, DMSO-*d₆*) δ 8.88 (d, J = 8.3 Hz, 1H), 7.85 (s, 1H), 7.37-7.30 (m, 4H), 7.04 (br, 2H), 5.30-5.13 (m, 2H), 4.25-4.16 (m, 1H), 2.34-2.30 (m, 4H), 2.40-2.25 (m, 4H), 2.20 (s, 3H), 1.54-1.45 (m 2H), 1.37-1.26 (m, 2H), 1.16 (d, J = 6.5 Hz, 3H), 0.88 (t, J = 7.3 Hz, 3H).

### Compound I-61

### 2-amino-4-(butylamino)-6-((4-(pyrrolidin-1-ylmethyl)thiazol-2-yl)methyl) pyrimido[4,5-d]pyridazin-5(6H)-one

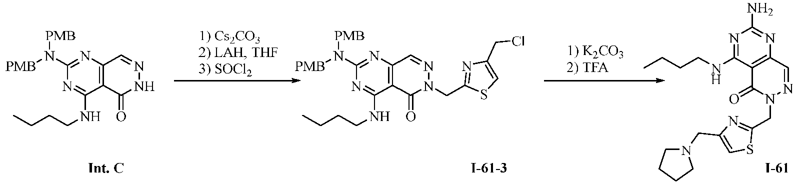

Compound I-61 was synthesized with reference to Compound I-1 by using Intermediate Int. C instead of Intermediate Int. A and methyl 2-(chloromethyl)thiazole-4-carboxylate instead of methyl 4-(bromomethyl) benzoate to obtain white solid compound I-61. MS: 415.2 [M+H]⁺.

### Compound I-62

### 2-amino-4-(butylamino)-6-((5-(pyrrolidin-1-ylmethyl)pyridin-2-yl)methyl) pyrimido[4,5-d]pyridazin-5(6H)-one

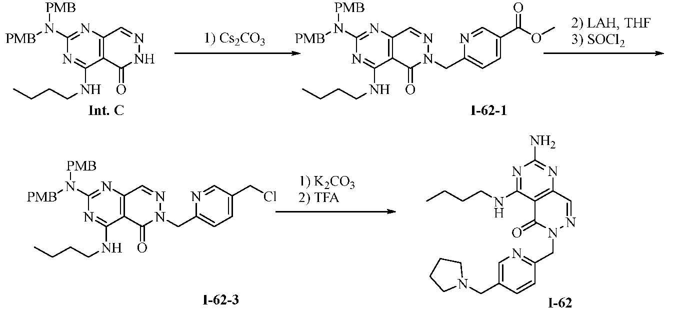

Compound I-62 was synthesized with reference to Compound I-1 by using Intermediate Int. C instead of Intermediate Int. A and methyl 6-(chloromethyl)nicotinate instead of methyl 4-(bromomethyl) benzoate to obtain white solid compound 1-62. MS: 409.2 [M+H]⁺.

### Compound I-63

### (R)-2-amino-4-(hexan-3-ylamino)-6-(4-(pyrrolidin-1-ylmethyl)benzyl)pyri mido[4,5-d]pyridazin-5(6H)-one

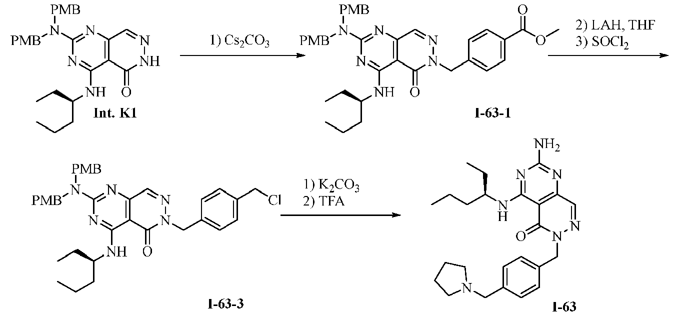

Compound I-63 was synthesized with reference to Compound I-1 by using Intermediate Int. K1 instead of Intermediate Int. A to obtain white solid compound 1-63. MS: 436.2 [M+H]⁺.¹H NMR (400 MHz, DMSO-*d₆*) δ 9.06 (s, 1H), 7.93 (s, 1H), 7.48 (d, J = 8.0 Hz, 2H), 7.44-7.32 (m, 4H), 5.26 (s, 2H), 4.32 (d, J = 5.7 Hz, 2H), 4.22-4.12 (m, 1H), 3.40-3.29 (m, 2H), 3.12-3.00 (m, 2H), 2.05-1.95 (m, 2H), 1.87-1.75 (m, 2H), 1.65-1.42 (m, 4H), 1.36-1.20 (m, 2H), 0.87 (q, J = 7.3 Hz, 6H).

### Compound I-64

### (R)-2-amino-6-(4-(4-(4-(2-hydroxyethyl)piperazine-1-carbonyl)benzyl)-4-( pentyl-2-amino)pyrimido[4,5-d]pyridazin-5(6H)-one

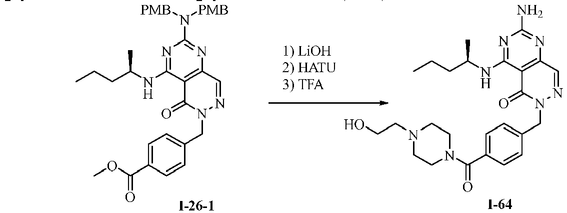

Compound I-64 was synthesized with reference to Compound I-60 by using 2-(piperazin-1-yl)ethan-1-ol instead of 1-methylpiperazine to obtain white solid compound 1-64. MS: 495.2 [M+H]⁺.¹H NMR (400 MHz, DMSO-*d₆*) δ 9.20 (d, J = 8.2 Hz, 1H), 8.00 (s, 1H), 7.69 (br, 2H), 7.47-7.35 (m, 4H), 5.38-5.21 (m, 2H), 4.30-4.19 (m, 1H), 3.76-3.68 (m, 2H), 3.62-3.30 (m, 4H), 3.23-3.16 (M, 2H), 3.6-3.05 (s, 4H), 1.59-1.47 (m, 2H), 1.40-1.25 (m, 2H), 1.19 (d, J = 6.5 Hz, 3H), 0.89 (t, J = 7.3 Hz, 3H).

### Compound I-66

### (S)-2-amino-4-((1-hydroxypentan-2-yl)amino)-6-(4-((4-methylpiperazin-1-yl)methyl)benzyl)pyrimido[4,5-d]pyridazin-5(6H)-one

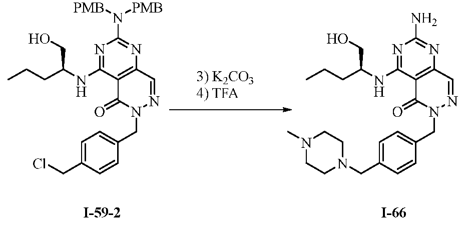

Compound I-66 was synthesized with reference to Compound I-59 by using 1-methylpiperazine instead of pyrrolidine to obtain white solid compound 1-66. MS: 467.2 [M+H]⁺.¹H NMR (400 MHz, DMSO-*d₆*) δ 9.02 (d, J = 8.7 Hz, 1H), 7.83 (s, 1H), 7.26-7.20 (m, 4H), 7.00 (br, 2H), 5.23 - 5.06 (m, 2H), 4.23-4.13 (m, 1H), 3.51 - 3.45 (m, 2H), 3.43 (s, 2H), 2.57 - 2.50 (m, 4H), 2.47 - 2.31 (m, 4H), 2.29 (s, 3H), 1.64 - 1.41 (m, 2H), 1.35 - 1.24 (m, 2H), 0.88 (t, J = 7.3 Hz, 3H).

### Compound I-67

### (S)-2-amino-4-((1-hydroxyhexan-2-yl)amino)-6-(4-(pyrrolidin-1-ylmethyl) benzyl)pyrimido[4,5-d]pyridazin-5(6H)-one

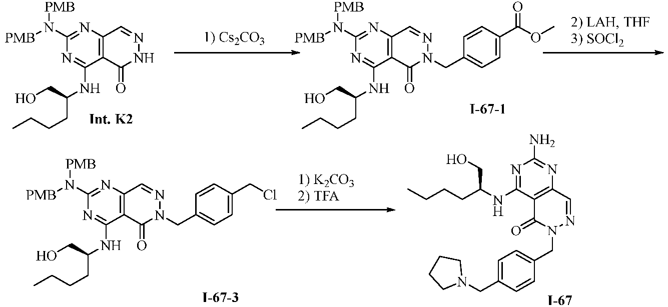

Compound I-67 was synthesized with reference to Compound I-1 by using Intermediate Int. K2 instead of Intermediate Int. A to obtain white solid compound 1-67. MS: 452.2 [M+H]⁺.¹H NMR (400 MHz, DMSO-*d₆*) δ 9.79 (s, 1H), 9.23 (s, 1H), 7.94 (s, 1H), 7.48 (d, J = 8.0 Hz, 2H), 7.44 - 7.34 (m, 3H), 5.26 (q, J = 14.9 Hz, 2H), 4.32 (d, J = 5.7 Hz, 2H), 4.19 (s, 2H), 3.55 - 3.44 (m, 2H), 3.41 - 3.30 (m, 2H), 3.15 - 3.01 (m, 2H), 2.11 - 1.94 (m, 2H), 1.91 - 1.76 (m, 2H), 1.71 - 1.57 (m, 1H), 1.56 - 1.43 (m, 1H), 1.36 - 1.21 (m, 4H), 0.89 - 0.80 (t, J = 3.2 Hz, 3H).

### Compound I-68

### (R)-2-amino-4-((1-hydroxypentan-2-yl)amino)-6-(4-(pyrrolidin-1-ylmethyl )benzyl)pyrimido[4,5-d]pyridazin-5(6H)-one

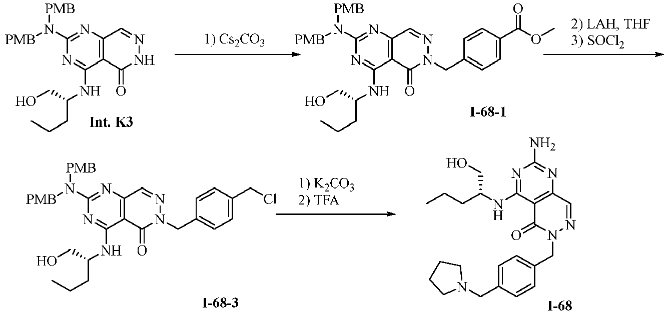

Compound I-68 was synthesized with reference to Compound I-1 by using Intermediate Int. K3 instead of Intermediate Int. A to obtain white solid compound I-68. MS: 438.2 [M+H]⁺.

### Compound I-69

### (S)-2-amino-4-((1-hydroxypentan-2-yl)amino)-6-(4-(4-methylpiperazine-1-carbonyl)benzyl)pyrimido[4,5-d]pyridazin-5(6H)-one

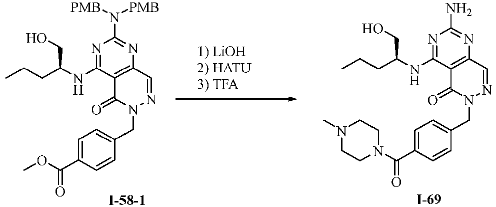

Compound I-69 was synthesized with reference to Compound I-58 to obtain white solid compound 1-69. MS: 481.2 [M+H]⁺.¹H NMR (400 MHz, DMSO-*d₆*) δ 7.98 (s, 1H), 7.47 (d, J = 8.2 Hz, 3H), 7.41 (d, J = 8.1 Hz, 2H), 5.34 (d, J = 15.0 Hz, 1H), 5.27 (d, J = 14.9 Hz, 1H), 4.24 (s, 2H), 3.52 (dq, J = 10.6, 5.5, 4.2 Hz, 4H), 3.43 - 3.31 (m, 2H), 3.10 (s, 2H), 2.84 (s, 3H), 2.54 (d, J = 2.0 Hz, 2H), 2.52 (d, J = 2.4 Hz, 2H), 1.57 (ddq, J = 36.0, 13.6, 7.9, 7.3 Hz, 2H), 1.35 (h, J = 7.3 Hz, 2H), 0.92 (t, J = 7.3 Hz, 3H).

### Compound I-70

### 6-(4-(3,6-diazabicyclo[3.1.1]heptane-3-carbonyl)benzyl)-2-amino-4-(((S)-1 -hydroxypentan-2-yl)amino)pyrimido[4,5-d]pyridazin-5(6H)-one

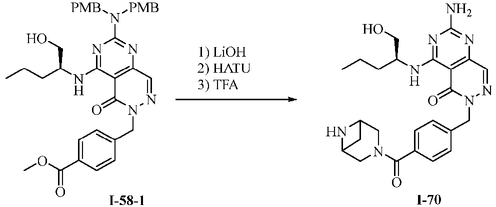

Compound I-70 was synthesized with reference to Compound I-58 to obtain white solid compound I-70. MS: 479.2 [M+H]⁺.¹H NMR (400 MHz, DMSO-*d₆*) δ 9.51 (s, 1H), 8.20 (s, 1H), 7.96 (d, J = 1.3 Hz, 1H), 7.49 (s, 1H), 7.45 (dd, J = 8.3, 1.5 Hz, 3H), 7.43 - 7.36 (m, 3H), 5.28 (q, J = 14.9 Hz, 2H), 4.40 (s, 1H), 4.03 (s, 1H), 3.90 (s, 1H), 3.81 (s, 1H), 3.73 (d, J = 12.6 Hz, 2H), 3.50 (q, J = 6.4, 5.4 Hz, 2H), 2.79 (d, J = 9.2 Hz, 1H), 1.82 (dd, J = 10.3, 5.4 Hz, 1H), 1.67 - 1.58 (m, 1H), 1.53 (s, 2H), 1.33 (p, J = 7.3 Hz, 2H), 0.92 - 0.86 (m, 3H).

### Compound I-71

### (S)-2-amino-4-((1-hydroxypentan-2-yl)amino)-6-(4-(piperazin-1-ylmethyl) benzyl)pyrimido[4,5-d]pyridazin-5(6H)-one

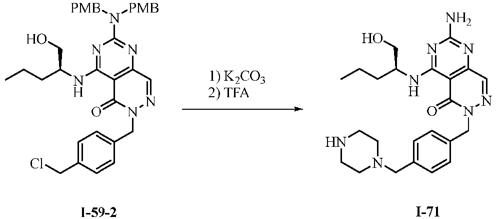

Compound I-71 was synthesized with reference to Compound I-59 to obtain white solid compound I-71. MS: 453.2 [M+H]⁺.¹H NMR (400 MHz, DMSO-*d₆*) δ 9.06 (d, J = 8.7 Hz, 1H), 8.80 (s, 2H), 7.87 (s, 1H), 7.30 (d, J = 1.7 Hz, 4H), 7.04 (s, 2H), 5.31 - 5.09 (m, 2H), 4.91 (s, 1H), 4.22 (tt, J = 8.6, 4.1 Hz, 1H), 3.52 (d, J = 12.8 Hz, 4H), 3.10 (t, J = 5.0 Hz, 4H), 2.57 (d, J = 5.3 Hz, 3H), 1.67 - 1.44 (m, 2H), 1.35 (p, J = 8.2, 6.6 Hz, 2H), 0.91 (t, J = 7.3 Hz, 3H).

### Determination of agonistic activity of the inventive compounds on human TLR7 or TLR8

The agonistic activity of the compounds of the present invention on TLR7 or TLR8 was evaluated using HEK-BlueTM cells stably expressing human TLR7 or TLR8. And the agonistic activity was assayed using their ability to induce the SEAP reporter gene under the control of the IFN-β minimal promoter fused to five NF-κB and ap-1 binding sites, specifically as follows:
HEK-Blue^{™} hTLR7 (Invivogen, 80,000 cells per well) or HEK-BlueTM hTLR8 (Invivogen, 60,000 cells per well) was added to a 96-well cell culture plate, the compound to be tested is added whereafter, and the final concentration of the compound(s) to be tested in the medium ranged from 0.001 to 36 µM, then incubated for 16~22 hours. SEAP levels in the cell culture medium were detected using HEK-Blue assay reagent Quanti-blue (Invivogen) according to the manufacturer's instructions. Neo2 Multifunctional Enzyme Labeler (Bio-tek) was used to determine the light absorption at 650 nm. GraphPad Prism was utilized to calculate the EC50, which is the half effective concentration of the drug.

**Table 1: EC₅₀ values of the compounds of the present invention for agonism of human TLR7/8**

| Compound No. | HEK-Blue TLR7 EC₅₀ (µM) | HEK-Blue TLR8 EC₅₀ (µM) | Compound No. | HEK-Blue TLR7 EC₅₀ (µM) | HEK-Blue TLR8 EC₅₀ (µM) |
|---|---|---|---|---|---|
| **I-1** | - | 0.107 | **I-2** | - | 0.102 |
| **I-3** | - | 0.615 | **I-4** | - | 0.403 |
| **I-5** | - | 0.149 | **I-7** | - | 0.153 |
| **I-8** | - | 0.229 | **I-9** | - | 0.183 |
| **I-10** | - | 0.569 | **I-11** | - | 0.267 |
| **I-12** | - | 0.185 | **I-13** | - | 0.549 |
| **I-14** | - | 0.365 | **I-15** | - | 0.326 |
| **I-16** | - | 0.354 | **I-17** | - | 0.368 |
| **I-18** | - | 0.218 | **I-19** | 0.922 | 0.149 |
| **I-21** | - | 0.388 | **I-23** | - | 0.222 |
| **I-24** | 0.634 | 0.194 | **I-25** | 0.631 | 0.147 |
| **I-26** | - | 0.322 | **I-27** | - | 0.286 |
| **I-39** | 0.641 | 0.181 | **I-41** | - | 0.101 |
| **I-40** | 0.9 | 0.083 | **I-42** | - | 0.074 |
| **I-47** | 0.357 | 0.07 | **I-48** | 0.921 | 0.203 |
| **I-49** | 0.964 | 0.073 | **I-50** | 0.77 | 0.157 |
| **I-51** | 0.892 | 0.141 | **I-52** | 0.785 | 0.125 |
| **I-43** | - | 0.233 | **I-44** | - | 0.112 |
| **I-53** | 0.597 | 0.175 | **I-55** | - | 0.169 |
| **I-56** | - | 0.07 | **I-57** | 0.407 | 0.107 |
| **I-58** | - | 0.073 | **I-59** | - | 0.009 |
| **I-60** | - | 0.192 | **I-62** | - | 0.589 |
| **I-63** | - | 0.534 | **I-66** | - | 0.023 |
| **I-67** | - | 0.051 | **I-69** | - | 0.041 |
| **I-70** | - | 0.02 | | - | |

Conclusion: The compounds of the present invention have good TLR7/8 agonistic activity.

### Pharmacokinetic (PK) evaluation in mice

Pharmacokinetic experiments were performed by MediSipia Pharmaceutical Technology (Shanghai) Co. Ltd. and the test animals were ICR mice (Shanghai Sipul-Bikai Laboratory Animal Co. Ltd.). The test compounds were administered to ICR mice via intravenous (iv) injection. The iv dose was 2 mg/kg and the solvent system was 100% Saline (0.9% saline). Animals fasted overnight (10-14 hours) before dosing and were given food 4 hours after dosing. Blood was collected into sodium heparin anticoagulant tubes at multiple time points (0.083, 0.25, 0.5, 1, 2, and 4 hours) after administration.

Test compounds were administered to ICR mice via gavage (po). The po was administered at a dose of 5 mg/kg and the solvent system was 100% Saline (0.9% saline). Animals fasted overnight (10-14 hours) before dosing and were given food 4 hours after dosing. Blood was collected into sodium heparin anticoagulation tubes at multiple time points (0.5, 1, 3, and 5 hours) after administration.

Test compound were administered to ICR mice subcutaneous administration (sc). The sc was administered at a dose of 5 mg/kg and the solvent system was 100% Saline (0.9% saline). Animals fasted overnight (10-14 hours) before dosing and were given food 4 hours after dosing. Blood was collected into sodium heparin anticoagulation tubes at multiple time points (0.5, 1, 3, and 5 h) after administration. Samples were processed, drug concentrations in plasma were analyzed by LC-MS/MS method, and pharmacokinetic parameters were calculated using Phoenix WinNonlin.

The results show that the compounds of the present invention have special pharmacokinetic properties and possess good druggability.

All documents referred to in this invention are cited as references in this application as if each document were cited individually as a reference. It is further to be understood that after reading the foregoing teachings of the present invention, a person skilled in the art may make various alterations or modifications to the present invention, and these equivalent forms will likewise fall within the scope of the claims appended to this application.

## Claims

1. A compound of formula I, or a solvate, a prodrug, a metabolite, or a pharmaceutically acceptable salt thereof, 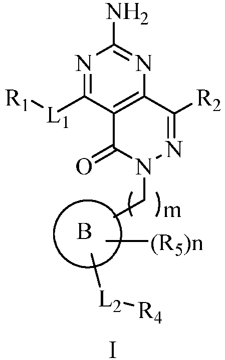 wherein:
L₁ is selected from the group consisting of: -O-, -NH-, -S-, -S(=O)- and -S(=O)₂-;
R₁ is selected from the group consisting of: H, C₁₋₁₂ alkyl, C₂₋₁₂ alkenyl, C₂₋₁₂ alkynyl, C₃₋₁₂ cycloalkyl and 4-12-membered heterocycloalkyl; wherein R₁ may be further substituted by one or more R^{a} substituents, and R^{a} is selected from the group consisting of: hydrogen, halogen, hydroxyl, cyano, C₁₋₆ alkyl, C₁₋₆ alkoxy, C₃₋₆ cycloalkyl, -NR^{a1}R^{a2}, -NHC(=O)-R^{a3}, and one or more R^{a4}-substituted 5-6-membered heteroaryl;
R^{a1}, R^{a2}, R^{a3} and R^{a4} are selected from the group consisting of C₁₋₆ alkyl, C₁₋₆ haloalkyl and C₃₋₆ cycloalkyl;
R^{a5} is selected from the group consisting of: C₁₋₂₄ alkyl, C₁₋₂₄ haloalkyl and C₁₋₂₄ heterocycloalkyl having 1-10 heteroatoms; wherein the heteroatoms are selected from one or more of NH, N, O and S;
R₂ is independently selected from the group consisting of: hydrogen, halogen, cyano, C₁₋₆ alkyl, C₁₋₆ alkoxy, C₃₋₆ cycloalkyl, or 4-6-membered heterocycloalkyl; wherein R₂ may be further substituted by one or more substituents selected from the group consisting of: halogen, hydroxy, cyano and amino;
m is 0, 1, 2, 3, 4, 5, 6, 7 or 8;
B is absent, or B is selected from the group consisting of: C₃₋₁₂ cycloalkyl, 4-12-membered heterocycloalkyl, C₆₋₁₂ aryl, 5-12-membered heteroaryl and 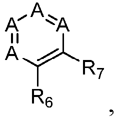
wherein each A is independently selected from C, CH and N, and R₆ and R₇ together with their attached carbon atoms form a C₄₋₇ cycloalkylidene or a C₄₋₇ heterocycloalkylidene, wherein one or more methylene groups in the C₄₋₇ cycloalkylidene or 4-7 membered heterocycloalkylidene may be each independently replaced by a carbonyl or a S(=O)₂; and heteroatoms in the 4-7 membered heterocycloalkylidene are selected from N, O and S, and the number of heteroatoms is from 1 to 3;
L₂ is selected from the group consisting of: none, -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O)-, -S(=O)- and -S(=O)₂-; wherein R^{b} and R^{c} are selected from the group consisting of: hydrogen, halogen, C₁₋₆ alkyl , C₃₋₆ cycloalkyl, 4-6 membered heterocycloalkyl and C₁₋₆ haloalkyl, R^{d} is selected from the group consisting of hydrogen, C₁₋₆ alkyl, C₃₋₆ cycloalkyl, 4-6 membered heterocycloalkyl, C₁₋₆ haloalkyl and hydroxyl-substituted C₁₋₆ alkyl, p is 0, 1, 2, 3, 4, 5, or 6, and q is 0 or 1;
R₄ is selected from the group consisting of: hydrogen, halogen, cyano, amino, hydroxy, C₁₋₁₂ alkyl, C₁₋₁₂ alkoxy, C₂₋₁₂ alkenyl, C₂₋₁₂ alkynyl, C₃₋₁₂ cycloalkyl, 4-12 membered heterocycloalkyl, C₆₋₁₂ aryl, or 5-12 membered heteroaryl; and R₄ is optionally substituted by one or more R^{e} substituents, wherein R^{e} is selected from the group consisting of: hydrogen, halogen, hydroxy, carboxylic acid, amino, C₁₋₆ alkyl, one or more R^{e1} substituted C₁₋₆ alkyl, C₁₋₆ alkoxy, C₁₋₆ haloalkoxy, C₃₋₁₂ cycloalkyl, 4-12 heterocycloalkyl, C₆₋₁₂ aryl, 5-12 membered heteroaryl, -N(R^{e2}R^{e3}), -C(=O)O-R^{e2}, -C(=O)NH-R^{e2} and -S(=O)₂-R^{e2},
and when L₂ is absent and R₄ is H, B is selected from the group consisting of: C₃₋₁₂ cycloalkyl, 4-12 membered heterocycloalkyl, C₆₋₁₂ aryl, 5-12-membered heteroaryl and 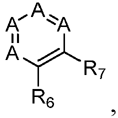 wherein each A is independently selected from C, CH and N, and R₆ and R₇ together with their attached carbon atoms form a C₄₋₇ cycloalkylidene or a C₄₋₇ heterocycloalkylidene;
R^{e1} is selected from the group consisting of: halogen and hydroxyl;
R^{e2} and R^{e3} are selected from the group consisting of: hydrogen, C₁₋₆ alkyl, C₃₋₆ cycloalkyl, C₁₋₆ haloalkyl and hydroxyl-substituted C₁₋₆ alkyl;
R₅ is selected from the group consisting of: halogen, hydroxyl, cyano, amino, C₁₋₆ alkyl, C₃₋₆ cycloalkyl, 4-6 membered heterocycloalkyl, C₁₋₆ haloalkyl, C₁₋₆ alkoxy, C₁₋₆ haloalkoxy, -C(=O)O-R^{f}, -C(=O)NH-R^{f}, and -S(=O)₂-R^{f}; wherein R^{f} is selected from the group consisting of: hydrogen, C₁₋₆ alkyl, C₃₋₆ cycloalkyl and C₁₋₆ haloalkyl;
n is 1, 2, 3, 4, 5 or 6;
wherein each heterocyclyl may be saturated or partially unsaturated (but do not have an aromatic structure), and in the heterocyclyl, the heteroatom is selected from N, O and S, and the number of heteroatoms is 1, 2, 3, or 4 (preferably 1 or 2); and in the heteroaryl, the heteroatom is selected from N, O and S, and the number of heteroatom is 1, 2, or 3.

2. The compound according to claim 1, or a solvate, a prodrug, a metabolite, or a pharmaceutically acceptable salt thereof, wherein L₁ is selected from the group consisting of: -O-, -NH- and -S-.

3. The compound according to claim 1, or a solvate, a prodrug, a metabolite, or a pharmaceutically acceptable salt thereof, wherein R₁ is selected from the group consisting of: C₁₋₁₂ alkyl, C₂₋₁₂ alkenyl, C₂₋₁₂ alkynyl, C₃₋₁₂ cycloalkyl and 4-12 membered heterocycloalkyl; and R₁ may be further substituted by one or more R^{a} substituents, and R^{a} is selected from the group consisting of halogen, hydroxyl, cyano, C₁₋₆ alkyl, C₁₋₆ alkoxy and C₃₋₆ cycloalkyl.

4. The compound according to claim 1, or a solvate, a prodrug, a metabolite, or a pharmaceutically acceptable salt thereof, wherein R₁ is selected from the group consisting of H, C₁₋₈ alkyl, C₁₋₈ alkoxy, C₂₋₈ alkenyl, C₂₋₈ alkynyl, C₃₋₈ cycloalkyl and 4-8 membered heterocycloalkyl; wherein R₁ may be further substituted by one or more R^{a} substituents, and R^{a} is selected from the group consisting of: hydrogen, halogen, hydroxyl, cyano, C₁₋₄ alkyl and C₁₋₄ alkoxy.

5. The compound according to claim 1, or a solvate, a prodrug, a metabolite, or a pharmaceutically acceptable salt thereof, wherein B is absent, or B is selected from the group consisting of: C₃₋₈ cycloalkyl, 4-7 membered heterocyclyl, C₆₋₁₀ aryl and

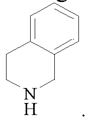

6. The compound according to claim 1, or a solvate, a prodrug, a metabolite, or a pharmaceutically acceptable salt thereof, wherein L₂ is selected from the group consisting of: -(CR^{b}R^{c})ₚ-(NR^{d})_{q}-, -O-, -S-, -(CR^{b}R^{c})ₚ-C(=O)-, -(CR^{b}R^{c})ₚ-C(=O)NH-, -(CR^{b}R^{c})ₚ-NHC(=O)-, -S(=O)- and-S(=O)₂-; wherein R^{b} and R^{c} are selected from the group consisting of: hydrogen, halogen and C₁₋₆ alkyl; R^{d} is hydrogen or C₁₋₆ alkyl; p is 0, 1, 2 or 3 and q is 0 or 1.

7. The compound according to claim **1,** or a solvate, a prodrug, a metabolite, or a pharmaceutically acceptable salt thereof, wherein R₄ is selected from the group consisting of: hydrogen, halogen, amino, hydroxyl, C₁₋₁₂ alkyl, C₁₋₁₂ alkoxy, C₂₋₁₂ alkenyl, C₂₋₁₂ alkynyl, C₃₋₁₀ cycloalkyl, 4-10 membered heterocycloalkyl, C₆₋₁₂ aryl, and 5-12 membered-heteroaryl; and R₄ may be optionally substituted by one or more R^{e} substituents; wherein R^{e} is selected from the group consisting of: hydrogen, halogen, hydroxyl, carboxylic acid, amino, C₁₋₆ alkyl, one or more R^{e1}-substituted C₁₋₆ alkyl, C₁₋₆ alkoxy, C₁₋₆ haloalkoxy, C₃₋₈ cycloalkyl, 4-7 membered heterocycloalkyl, phenyl, 5-7 membered heteroaryl, -N(R^{e2}R^{e3}); wherein R^{e1} is selected from the group consisting of halogen and hydroxyl; R^{e2} and R^{e3} are selected from the group consisting of hydrogen, C₁₋₆ alkyl, C₃₋₆ cycloalkyl, C₁₋₆ haloalkyl and hydroxy-substituted C₁₋₆ alkyl.

8. The compound according to claim 1, or a solvate, a prodrug, a metabolite, or a pharmaceutically acceptable salt thereof, wherein R₅ is selected from the group consisting of: halogen, hydroxyl, cyano, amino, C₁₋₆ alkyl, C₁₋₆ alkoxy and C₃₋₆ cycloalkyl.

9. The compound according to claim 1, or a solvate, a prodrug, a metabolite, or a pharmaceutically acceptable salt thereof, wherein the compound of formula I is selected from the group consisting of:

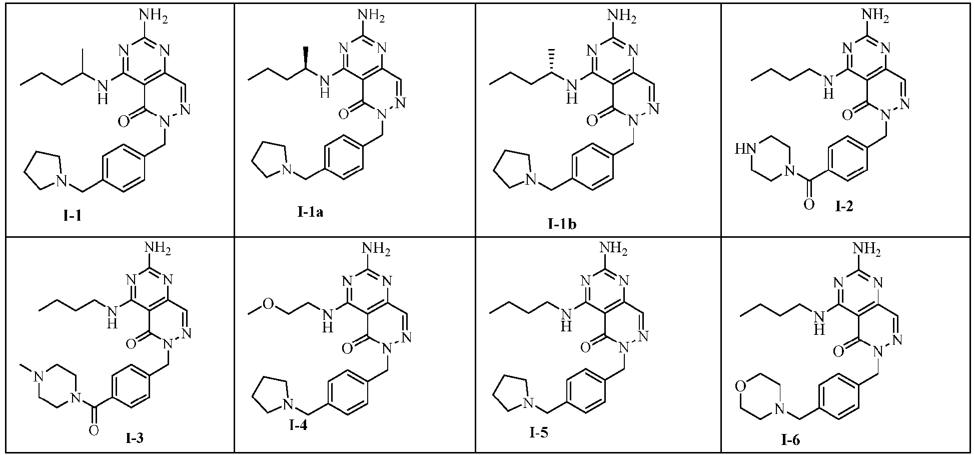

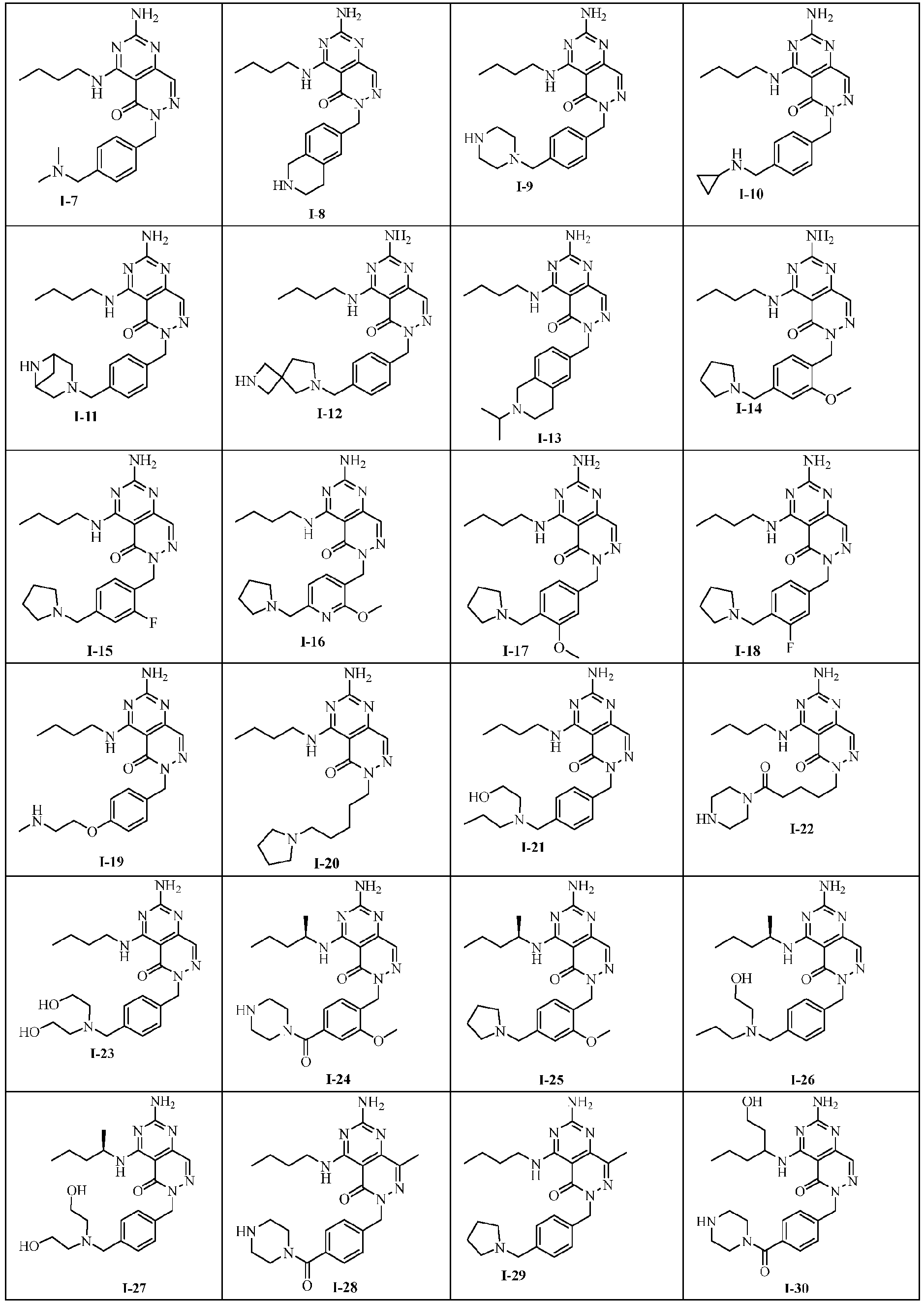

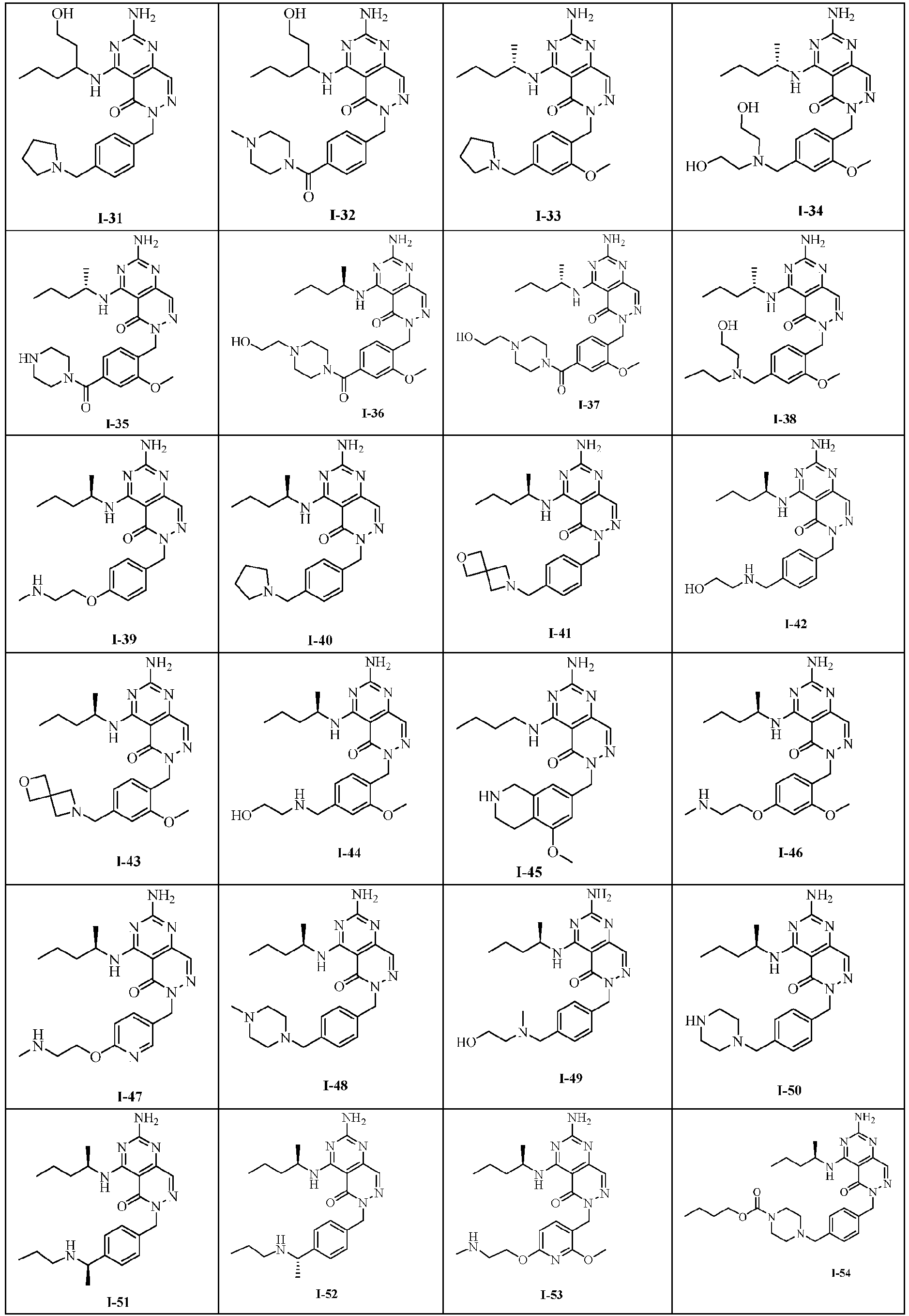

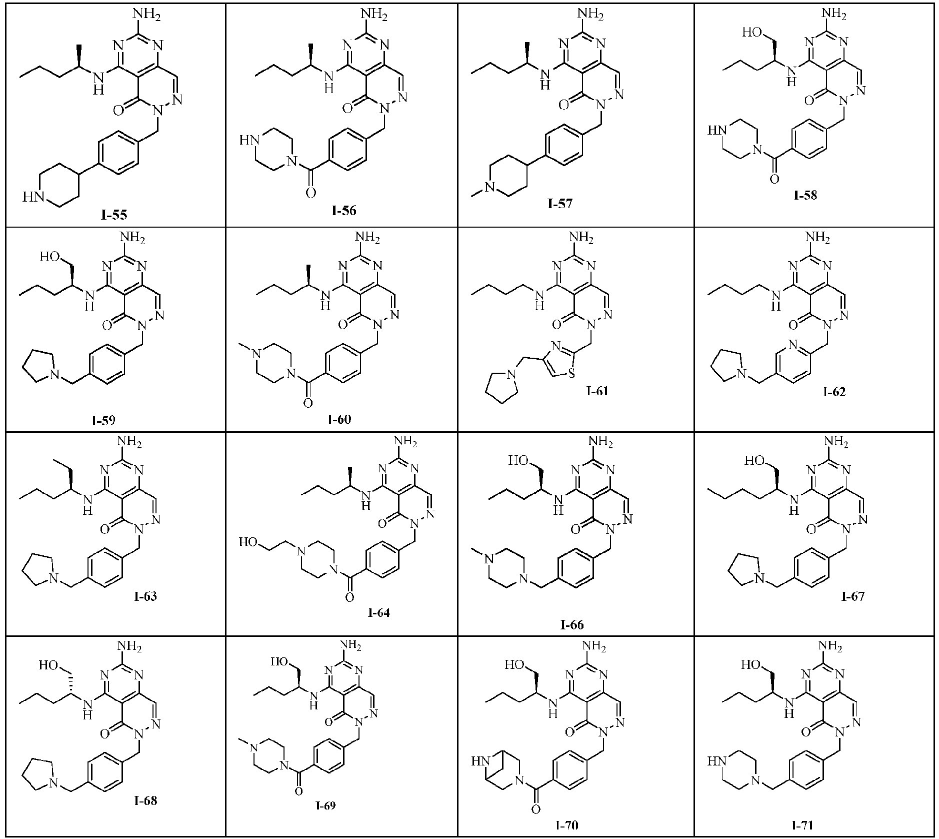

10. A pharmaceutical composition, comprising: one or more of the compound of formula I according to claim 1, a pharmaceutically usable salt thereof, a racemate, an R-isomer, an S-isomer, or a mixture thereof, and one or more pharmaceutically usable carriers, excipients, adjuvants, accessories and/or diluents.

11. Use of a compound of formula I according to claim 1, a pharmaceutically usable salt thereof, a racemate, an R-isomer, an S-isomer, or a mixture thereof in the preparation of a pharmaceutical compositions for the treatment or prevention of tumors or infections caused by viruses.
